# EUROPEAN PATENT APPLICATION

(11) **EP 4 147 723 A1**
(43) Date of publication of application: **15.03.2023**
(21) Application number: 21196945.6
(22) Date of filing: 15.09.2021
(51) Int. Cl.: A61K 47/55, A61K 47/54, A61P 25/28

(54) **BIFUNCTIONAL MOLECULES AS PROTEASOME STIMULATORS FOR IMPROVED TARGETED PROTEIN DEGRADATION, AND THEIR USES AS TARGETED BOOSTING DEGRADERS (TARBODS)**

(30) Priority: 09.09.2021 US 202163242235 P
(71) Applicant: Booster Therapeutics GmbH, 10117 Berlin (DE)
(72) Inventor: Feleciano, Diogo R., 13189 Berlin (DE); Eckstein, Jens W., Cambridge, 02138 (US); Trader, Darci J., West Lafayette, 47906 (US)
(74) Representative: Krauss, Jan

(57) **Abstract**

The present invention relates to molecules, pharmaceutical compositions and methods of use for the treatment and/or prevention of a disease or condition caused by an insufficient degradation of proteins by the proteasome system. The present invention particularly relates to a bispecific binding molecule, wherein said compound is an effective stimulator of the 20S core particle (CP) of the proteasome.

## Description

The present invention relates to molecules, pharmaceutical compositions and methods of use for the treatment and/or prevention of a disease or condition caused by an insufficient degradation of proteins by the proteasome system. The present invention particularly relates to a bispecific binding molecule, wherein said compound is an effective stimulator of the 20S core particle (CP) of the proteasome.

### Background of the invention

Targeted protein degradation (TPD) offers a novel therapeutic alternative by inducing the depletion or reduction of a disease-causing protein via hijacking the endogenous protein degradation machineries. TPD has the potential to target the undruggable proteome that limits current drug discovery efforts, as only a binder is required to recruit the target protein for degradation rather than high affinity inhibitors.

The major protein degradation pathway in cells is the ubiquitin-proteasome system (UPS). This system involves a network of proteins to polyubiquitinate and degrade protein substrates. Proteins are tagged for degradation with a small protein called ubiquitin. The tagging reaction is catalyzed by enzymes called ubiquitin ligases. Once a protein is tagged with a single ubiquitin molecule, this is a signal to other ligases to attach additional ubiquitin molecules. The result is a polyubiquitin chain that is bound by the proteasome, allowing it to degrade the tagged protein.

The degradation process is performed by the 26S proteasome, which is comprised of a 19S regulatory particle (19S RP) and a 20S core particle (20S CP). The 20S CP is responsible for the hydrolysis activity, degrading proteins into shorter peptides, and is regulated by the 19S RP, which recognizes ubiquitinated substrates, removes ubiquitin, and coordinates the movement of the substrate into the catalytic core particle for degradation.

The 20S CP alone can accept and degrade proteins in a ubiquitin-independent system (UIPS). In this case, proteins are not ubiquitinated and must be disordered enough to enter the catalytic core without being denatured by the 19S RP. The UIPS has been shown to play an important role in the degradation of oxidatively damaged proteins during times of cellular stress.

Aging is a natural process accompanied by a progressive accumulation of damage in all constituent macromolecules (nucleic acids, lipids and proteins). Accumulation of damage in proteins leads to failure of proteostasis (or vice versa) due to increased levels of unfolded, misfolded or aggregated proteins and, in turn, to aging and/or age-related diseases.

The proteasome and the lysosome have been shown to dysfunction during aging and age-related diseases, Parkinson's, and Alzheimer's disease. Regarding the proteasome, it is well established that it can be activated either through genetic manipulation or through treatment with natural or chemical compounds that eventually result to extension of lifespan or deceleration of the progression of age-related diseases. Stimulation of the 20S CP has recently been shown to be promoted by small molecules, such as AM-404 (AM), an AM-404 derivative (TRC-1), ursolic acid (UA), and miconazole (MO), leading to a more rapid degradation of disordered proteins (see, for example, Coleman, Rachel A, et al.; Protein degradation profile reveals dynamic nature of 20S proteasome small molecule stimulation. 2021, 636-644, RSC Chem. Biol. 2, doi:10.1039/D0CB00191K). In biochemical proteasome activity assays, MO and AM404 displayed the greatest stimulatory activity, increasing 20S CP activity more than 400% (at 25 µM) over the basal level control.

Njomen and Tepe (in: Proteasome Activation as a New Therapeutic Approach To Target Proteotoxic Disorders. J Med Chem. 2019;62(14):6469-6481. doi:10.1021/acs.jmedchem.9b00101) review current approaches, genetic manipulation, posttranslational modification, and small molecule proteasome agonists used to increase proteasome activity, challenges facing the field, and applications beyond aging and neurodegenerative diseases. Molecules that interact with either the 20S or both the 20S and 26S proteasome, function through two main mechanisms, namely: (1) *Gate-openers*: Molecules that promote substrate entry into the catalytic pocket through allosteric interactions that induce gate-opening in the alpha ring of the 20S proteasome. (2) *Stimulators*: Molecules that promote 20S and/or 26S-mediated degradation through allosteric interactions that enhance substrate binding and/or degradation in one or more catalytic sites. Among the first reported 20S agonists is the triterpenoid, betulinic acid, which was reported to specifically enhance the CT-L activity of the 20S proteasome. Unfortunately, chemical modifications to improve activity resulted in proteasome inhibitors with complicated structure activity relationships (SAR).

WO 2021/034627A1 relates to series compounds and methods of use for the treatment of a disease caused by abnormal regulation of the ubiquitin-proteasome system (UPS), and wherein said compound is an effective stimulator of the 20S core particle (CP) of the UPS.

Technologies that induce targeted protein degradation by small molecules have been developed recently. Chimeric small molecules such as Proteolysis Targeting Chimeras (PROTACs) and Specific and Non-genetic IAP-dependent Protein Erasers (SNIPERs), and E3 modulators such as thalidomides, hijack the cellular machinery for ubiquitylation, and the ubiquitylated proteins are subjected to proteasomal degradation. This has motivated drug development in industry and academia because "undruggable targets" can now be degraded by targeted protein degradation (Naito M, Ohoka N, Shibata N, Tsukumo Y. Targeted Protein Degradation by Chimeric Small Molecules, PROTACs and SNIPERs. Front Chem. 2019 Dec 10;7:849. doi: 10.3389/fchem.2019.00849. PMID: 31921772; PMCID: PMC6914816). PROTACs consist of a target protein ligand connected via a short linker to an E3 ligand, allowing PROTACs to function as bridging compounds that bring an E3 into proximity with specific cellular proteins. The juxtaposition of the E3 complex and target protein facilitates the processive transfer of ubiquitin from the E3 complex to the target protein, thereby tagging the protein for degradation via the proteasome.

WO 2020/041331A1 discloses such proteolysis targeting chimeric (protac) compound with e3 ubiquitin ligase binding activity and targeting alpha-synuclein protein for treating neurodegenerative diseases. Disclosed are bifunctional compounds, which contain on one end a Von Hippel-Lindau, cereblon, Inhibitors of Apotosis Proteins or mouse double-minute homolog 2 ligand which binds to the respective E3 ubiquitin ligase and on the other end a moiety which binds the target protein. The target protein is placed in proximity to the ubiquitin ligase to effect degradation (and inhibition) of target protein. Diseases or disorders to be treated are alpha-synucleinopathies or neurodegenerative diseases associated with alpha-synuclein accumulation and aggregation, such as e.g., Parkinson Disease, Alzheimer's Disease, dementia, dementia with Lewy bodies or multiple system atrophy, in particular Parkinson's Disease.

While empiric guidelines like the rule-of-five (C. A. Lipinski, et al., Adv. Drug Delivery Rev. 1997, 23, 3) for the optimization of traditional orally available small molecules inhibitors have been developed and refined over the past decades, optimizing the bioavailability for large molecules like PROTAC is still in its infancy. Optimizing a beyond rule-of-five compound with a molecular weight most likely between 700 and 1200 g mol^{―1} for clinical applications is an enormous challenge as many principles established for small molecule inhibitors do not translate to larger molecules. With only very few design guidelines available PROTAC optimization is still no straight forward process.

The development of more effective and selective molecules mediating proteasome degradation of undesired proteins is needed if they are to become useful in the treatment and/or prevention of diseases where undesired proteins and protein accumulation plays a role. It is an object of the present invention to provide effective agents that can be used for the prevention and treatment of such conditions and diseases that can be treated/prevented by inducing and/or stimulating proteasome degradation, in particular age-related diseases, such as neurological diseases. Other objects and advantages will become apparent to the person of skill when studying the present description of the present invention.

In a first aspect of the present invention, the above object is solved by a molecule improving the degradation of a proteinaceous target molecule by the proteasome of a cell, according to the general formula

B―L―S

wherein B indicates at least one binding moiety binding or promoting binding to the target molecule, S indicates at least one moiety stimulating proteasome degradation, and L indicates at least one linker moiety suitably linking said at least one binding moiety with said at least one moiety stimulating proteasome degradation, and pharmaceutically acceptable salts thereof.

The molecules as described herein were found to be surprisingly highly effective proteasome inducers/stimulators in order to specifically degrade target molecules. The molecules as described herein were found to have a synergistic effect on the proteasome activity, and/or when degrading the target molecules. The present inventors thus provide molecules for the induction and/or stimulation of proteasome in diseases and undesired conditions related to an accumulated undesired protein, in particular an accumulated pathological protein in a cell of a mammalian patient or subject, like a human. In the case of a molecule represented by the general structure according to the present invention, the proteasome of the target molecule is effectively induced and/or further stimulated in an ubiquitin-dependent and/or independent manner. Preferably, the molecule according to the present invention is bispecific.

The present invention specifically relates to the technology herein designated as TARBOD (TARgeted BOosting Degrader).

Particularly preferred is a molecule according to the present invention that is selected from the group of the compounds according to any one of formulae I to XXVIII, and pharmaceutically acceptable salts thereof.

In a second aspect of the present invention, the above object is solved by a method for producing a molecule according to the present invention, comprising suitably linking the at least one binding moiety (B) to the at least one moiety stimulating proteasome degradation (S) through the at least one linker moiety (L). In a preferred embodiment, the method further comprises the step of identifying the at least one binding moiety (B) and/or the at least one moiety stimulating proteasome degradation (S) before the linking thereof to the at least one linker moiety (L), i.e., involves a pre-selection of suitable components before the chemical and/or enzymatic production of the molecule according to the present invention.

In a third aspect of the present invention, the above object is solved by a pharmaceutical composition, comprising at least one molecule according to the present invention or a pharmaceutically acceptable salt thereof, together with at least one pharmaceutically acceptable carrier or buffer.

In a fourth aspect of the present invention, the above object is solved by the molecule according to the present invention or the pharmaceutical composition according to the present invention for use in the prevention and/or treatment of diseases or conditions in a mammalian subject, such as a human. The diseases or conditions to be treated or prevented are preferably selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing.

In a fifth aspect of the present invention, the above object is solved by a method for treating or preventing a disease or condition in a mammalian subject, such as a human, comprising administering to a subject in need of said treatment or prevention an effective amount of the molecule according to the present invention or the pharmaceutical composition according to the present invention, wherein the disease or condition to be treated or prevented is selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing.

In a sixth aspect of the present invention, the above object is solved by the use of the molecule according to the present invention or the pharmaceutical composition according to the present invention for improving proteasome function preferably in a mammalian cell, such as a human cell.

In a seventh aspect of the present invention, the above object is solved by the use of the molecule according to the present invention or the pharmaceutical composition according to the present invention for removing an undesired proteinaceous target molecule preferably in a mammalian cell, such as a human cell.

The methods and uses of the present invention can be performed in vivo and/or in vitro.

It was surprisingly found that the molecules according to the present invention as a proteasome inducer and in particular booster (i.e., having a synergistic effect) were effective and exhibit many advantages compared to other proteasome-related treatment options, such as, for example protacs. In comparison with prior art molecules as tested (including data not shown), an improvement of the present invention lies in the unexpected observation that the molecules as described herein are highly effective proteasome stimulators/inducers (see Examples below). Based on these results it is evident that these molecules will be active in target molecule-related diseases as disclosed herein. Furthermore, specific activities in models of diseases involving or caused by an undesired proteinaceous target molecule, for example caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's Disease, Alzheimer's Disease, dementia, dementia with Lewy bodies, multiple system atrophy, cancer, breast cancer, prostate cancer, cancer tumor metastasis, and ageing are anticipated. By way of an exemplary application, the molecules according to Formula I to XXVIII were shown to be surprisingly effective, e.g., synergistically effective (see Examples below).

The present invention generally provides molecules that improve - and even boost - the degradation of a proteinaceous target molecule by the proteasome of a cell. According to the invention, a molecule comprises at least the parts B, L, and S that are complexed and/or combined according to the formula B - L - S.

The components as indicated can thus be chemically bound and/or form a complex, i.e., a molecular entity formed by loose association involving two or more of the components based on other physical interactions than covalent bonds, such as charges, atomic interactions, or the like.

Surprisingly, the molecules according to the invention, such as, for example, according to Formula I to XXVIII were shown to be surprisingly effective inducers/stimulators of the proteasome, and even synergistically effective (boosters). Without wanting to be bound by theory, it appears that the strong effect is at least in part caused or related to the spatial arrangement of the three components B, L, and S, in particular the rather close molecular distance between B and S, as primarily controlled by the linker L, e.g., in the form of a (PEG)₃₋₅ structure (see examples, in particular Formula I to XXVIII). It seems that the longer the linker (such as a (PEG)₅ structure) the larger is the stimulation.

L indicates at least one linker moiety suitably linking said at least one binding moiety with said at least one moiety stimulating proteasome degradation, and L is preferably selected from the group consisting of a branched or unbranched chemical linker compound, a branched or unbranched linker amino acid sequence, a cleavable branched or unbranched linker compound, and a labelled branched or unbranched linker compound, in particular a polyethylene linker sequence, such as (PEG)₃.

Linkers based on poly(ethylene glycol) (PEG) are the most common types for chemical cross-linking of biomolecules, and therefore are preferred. Alternatives are XTEN, PAS, and GQAP amino acid linkers that are used as "recombinant PEG linkers" for cross-linking biomolecules, via introduction of reactive residues along the sequence (Thomas Kjeldsen, et al. Dually Reactive Long Recombinant Linkers for Bioconjugations as an Alternative to PEG ACS Omega 2020 5 (31), 19827-19833 DOI: 10.1021/acsomega.0c02712; and Podust, V. N.; et al. Extension of in vivo half-life of biologically active molecules by XTEN protein polymers. J. Controlled Release 2016, 240, 52- 66, DOI: 10.1016/j.jconrel.2015.10.038 ). Another alternative is the conjugation with polysarcosine (PSar) (Yali Hu, et al. Polysarcosine as an Alternative to PEG for Therapeutic Protein Conjugation Bioconjugate Chemistry 2018 29 (7), 2232-2238 DOI: 10.1021/acs.bioconjchem.8b00237). A suitable linker is preferably selected based on the size/distance requirements between B and S as mentioned above, and should show little or no immunogenicity *in vivo.* L can be more preferably selected from between (PEG)₂ to (PEG)₅ and branched derivatives thereof.

In the above formula, B indicates at least one binding moiety binding or promoting binding to the target molecule. Thus, the purpose of B is generally to bind or attach to a target molecule which is then (more efficiently compared to a normal introduction) introduced into the proteasomal degradation process. Target molecules in the context of the present invention are generally any undesired molecules that may be reduced or removed from the cell by proteasomal degradation. Preferably, therefore, target molecules are proteinaceous molecules that relate to or cause an undesired disease or condition, and molecules the removal of which provides a health benefit to the respective subject or patient. More preferred examples of target molecules are selected from an accumulated pathological protein, prions, beta-amyloid, tau, alpha-synuclein, estrogen receptor alpha (ERα), androgen receptor (AR), huntingtin, ataxin, methionine aminopeptidase-1 (MetAP-2), cellular retinoic acid-binding protein-I, cellular retinoic acid-binding protein-II (CRABP-II), alpha 1 antitrypsin, rhodopsin, crystallin, transthyretin, amyloid, cystic fibrosis transmembrane conductance regulator (CTFR), superoxide dismutase 1 (SOD1), transactive response DNA binding protein 43 kDa (TDP-43), fused in sarcoma (FUS), and islet amyloid polypeptide (IAPP), and preferably wherein the target molecule is selected from an extracellular protein.

In the context of the present invention, the at least one binding moiety (B) can be any suitable molecule binding or promoting binding to the target molecule. Respective binding moieties are known to the person of skill, and are disclosed in the literature. Preferred is a binding moiety from the group consisting of an antibody that is specific for the target molecule as above or a specific binding fragment thereof, a proteinaceous group specifically binding to the target molecule, a specifically binding peptide, a natural or non-natural binding nucleic acid, and a small molecule specifically binding to said target molecule, and specific examples are amyvid (florbetapir 18F), tauvid (flortaucipir 18F), and thioflavin.

In the context of the present invention, in the above formula, S indicates at least one binding moiety stimulating proteasome degradation (S). Respective compounds and moieties are known to the person of skill, and are also disclosed in the literature (Trader DJ; et al. Establishment of a Suite of Assays That Support the Discovery of Proteasome Stimulators. Biochim. Biophys. Acta, Gen. Subj 2017, 1861, 892-899; and Coleman and Trader RSC Chem. Biol., 2021, 2, 636). Preferably, the stimulator is selected from the group consisting of a compound stimulating degradation in an ubiquitin-independent and/or dependent manner, a compound stimulating the 20S core particle (CP) of the proteasome, such as miconazole (MO), AM-404 (AM), AM-404 derivative TRC-1, and ursolic acid (UA), and further derivatives thereof (see Coleman and Trader RSC Chem. Biol., 2021, 2, 636). Furthermore, compounds that enhance proteasome degradation in an ubiquitin-dependent manner by binding to the 19S RP can be incorporated into the bifunctional compounds. The proteasome stimulating moiety can have several characteristics, e.g., be a small molecule, or a peptide.

As mentioned above, the molecules according to the invention were shown to be surprisingly effective inducers/stimulators of the proteasome, and even synergistically effective (boosters). Preferred is thus the molecule according to the present invention, wherein said molecule synergistically stimulates/improves the degradation of the proteinaceous target molecule as disclosed herein (see below).

The molecules according to the general formula according to the present invention in a preferred embodiment contain miconazole as a proteasome stimulator (Coleman and Trader RSC Chem. Biol., 2021, 2, 636), (PEG)₃ as a linker, and a specific target binder (here, thioflavin, or flortaucipir, or florbetapir). The compounds as described herein are designed to deliver a protein of interest to the proteasome for degradation while boosting proteasome activity.

The molecules according to the general formula according to the present invention can be modified in order to create derivatives of the molecules that are also included in the scope of the present invention. Said modification can take place in an additional preferred step of the methods of the invention as described herein, wherein, for example, after analyzing the degradation of a protein in the presence and absence of said compound as selected, said compound is further chemically modified as described herein, and analyzed again for its effect. Said "round of modification(s)" can be performed for one or several times in all the methods, in order to optimize the effect of the compound, for example, in order to improve its specificity for the target protein. In general, all parts of the molecule may be modified (i.e. B, S), with the linker (L) being less preferred.

This method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity. The modification can also be simulated in silico before additional tests are performed in order to confirm or validate the effect of the modified selected or screened compound from the first round of screening. Respective software programs are known in the art and readily available for the person of skill.

Modification can further be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The present invention also includes pharmaceutically acceptable salts of the molecules according to the present invention. The term "pharmaceutically acceptable salt" refers to a pharmaceutically acceptable organic or inorganic salt of the compound of the invention. This may include addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Exemplary salts also include oxalate, chloride, bromide, iodide, bisulphate, acid phosphate, isonicotinate, salicylate, acid citrate, oleate, tannate, pantothenate, bitartrate, ascorbate, gentisinate, gluconate, glucuronate, saccharate, formate, benzoate, glutamate, ethanesulfonate, and benzenesulfonate salts. For other examples of pharmaceutically acceptable salts, reference can be made to Gould (1986, Int J Pharm 33: 201-217).

Most preferred is the molecule according to the present invention, which is selected from the group of the compounds according to any one of formulae I to XXVIII, and pharmaceutically acceptable salts and derivatives thereof as follows.

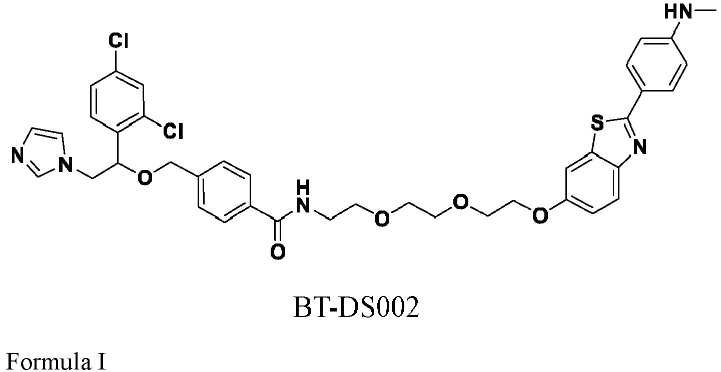

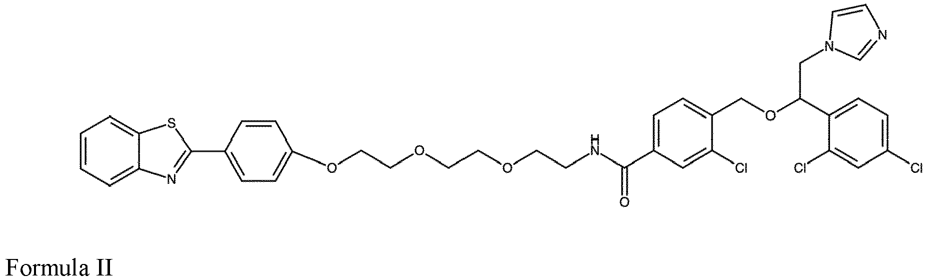

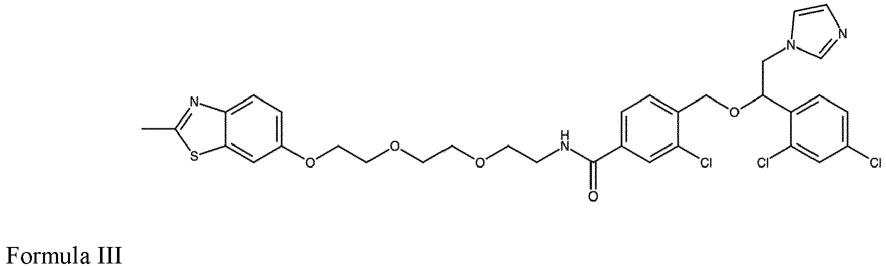

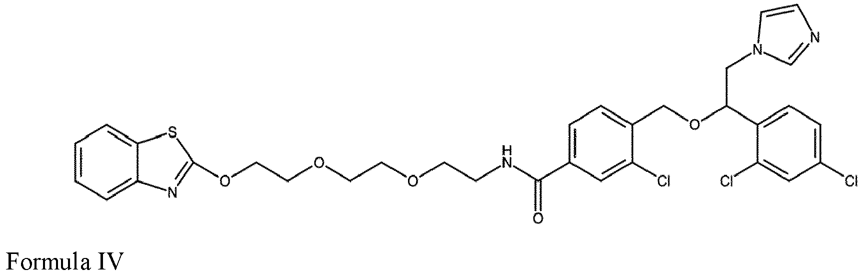

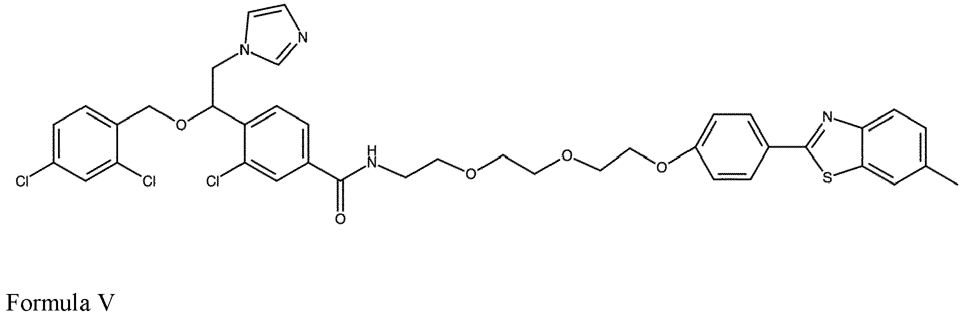

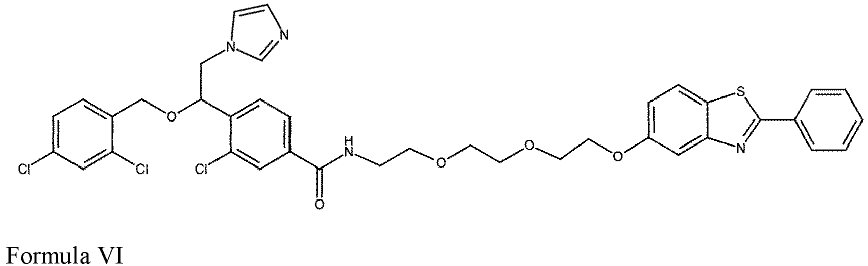

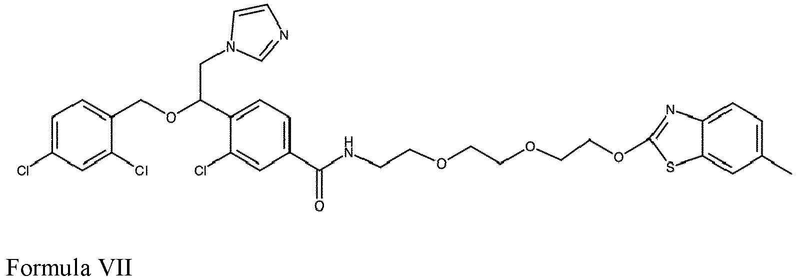

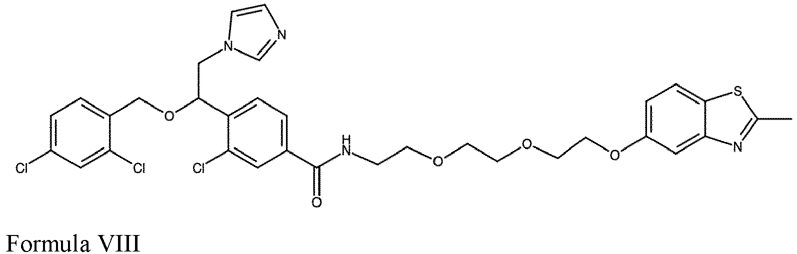

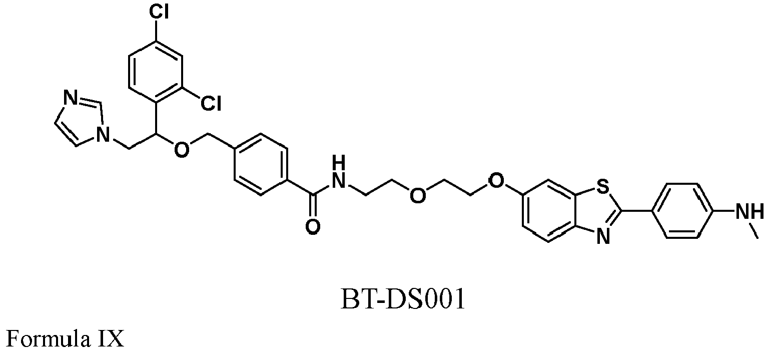

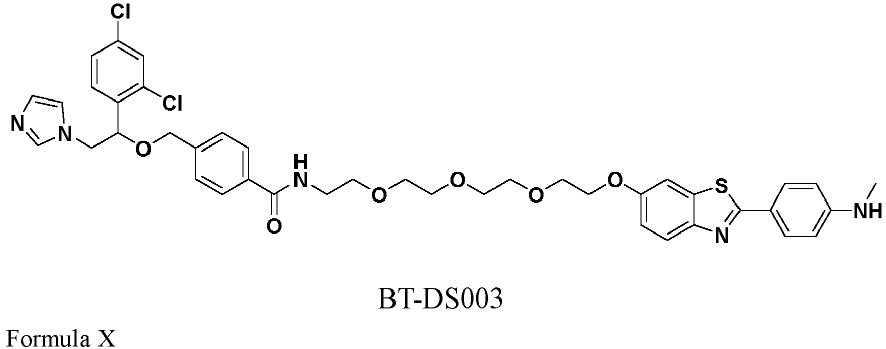

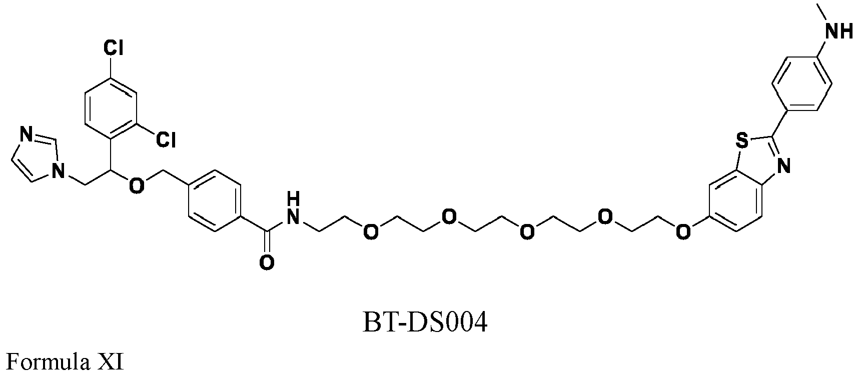

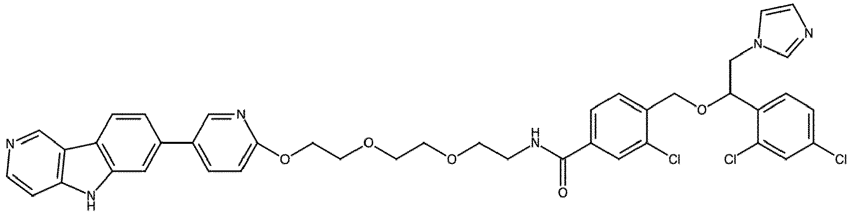

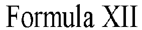

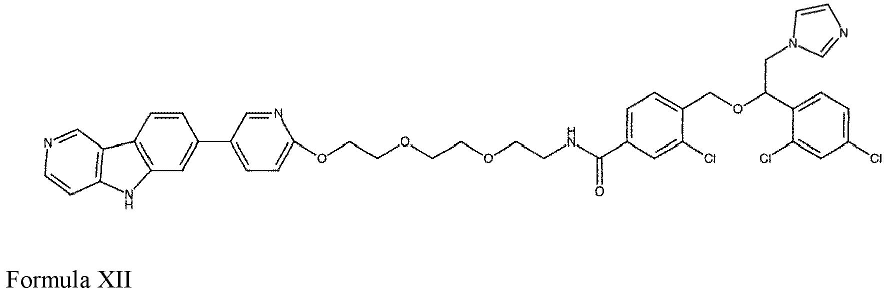

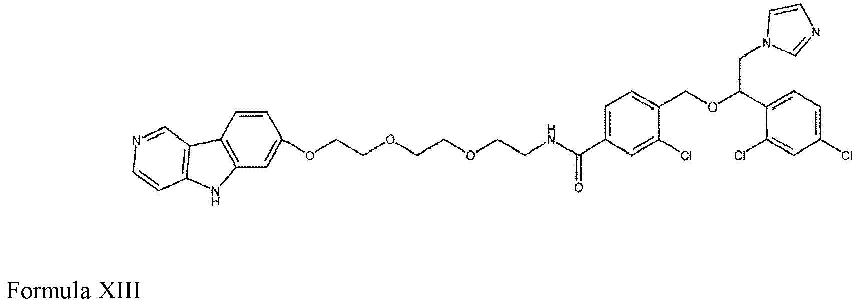

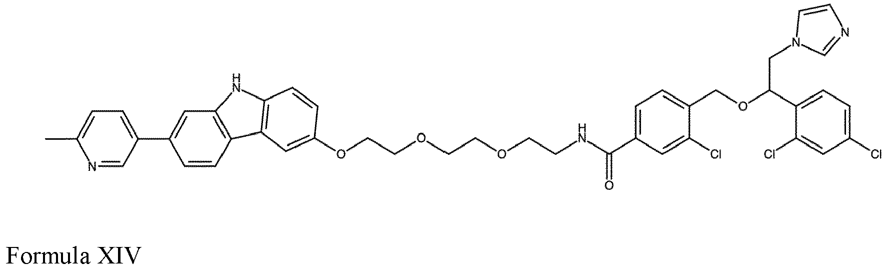

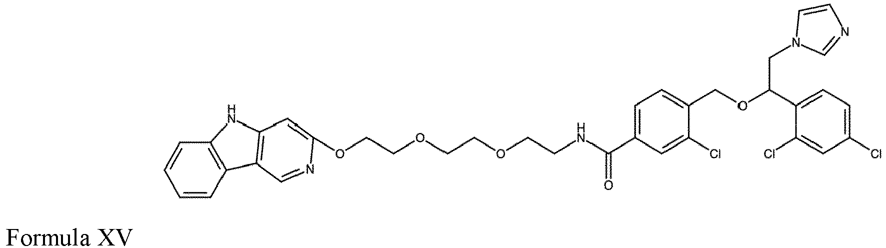

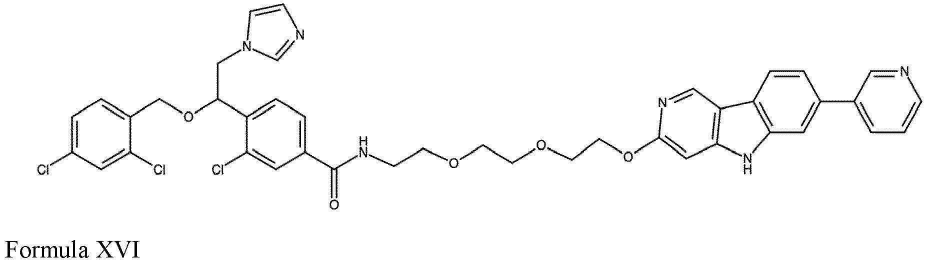

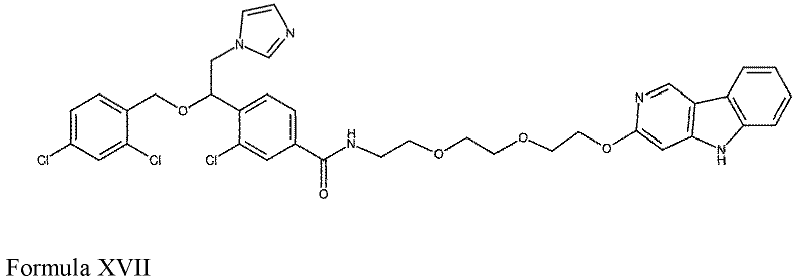

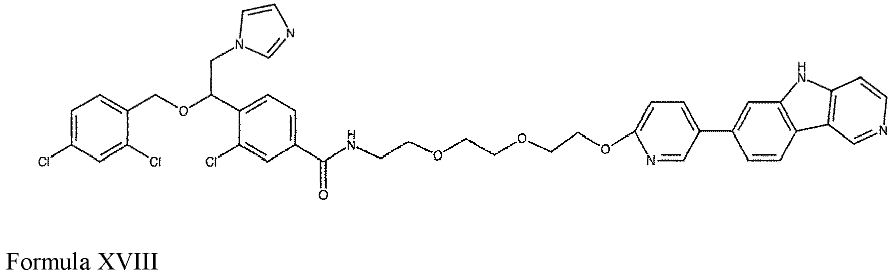

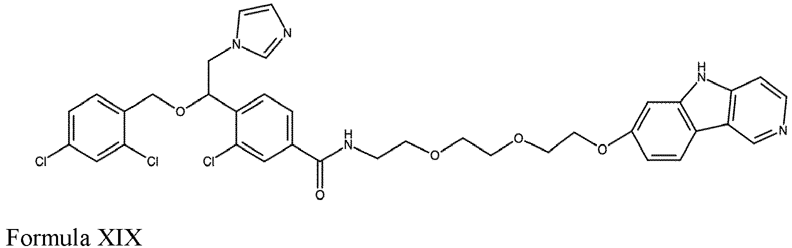

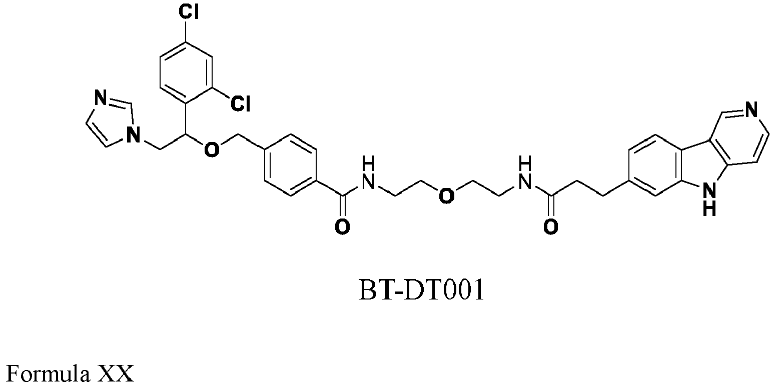

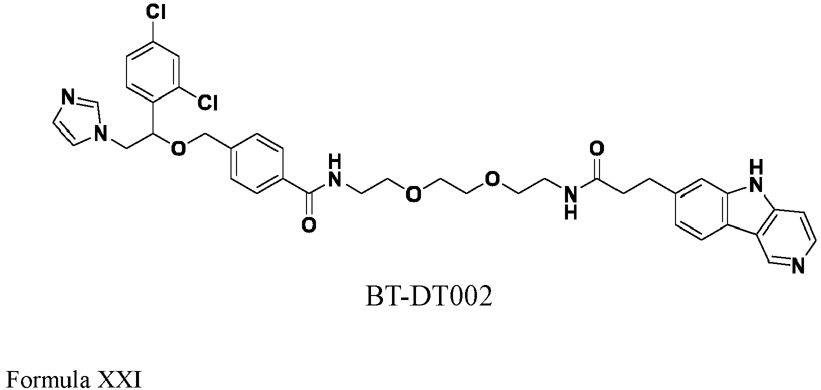

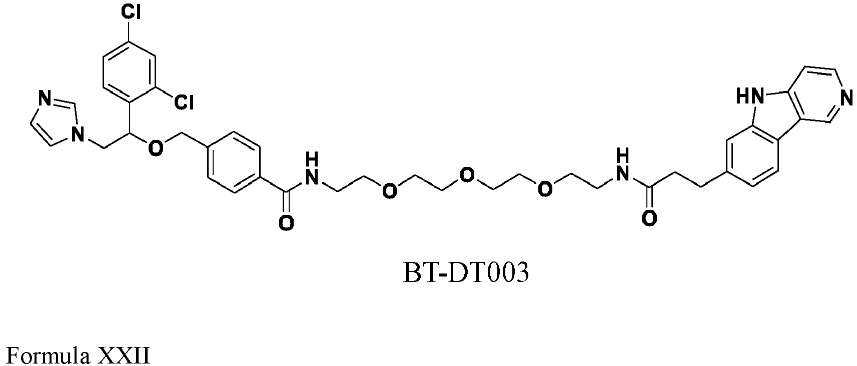

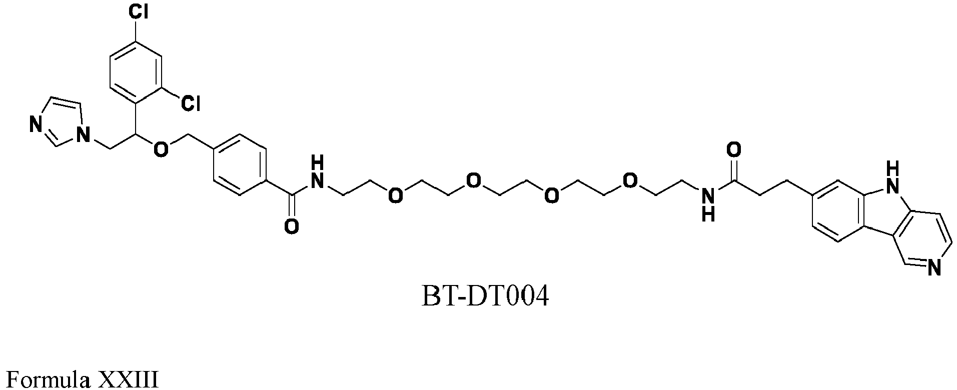

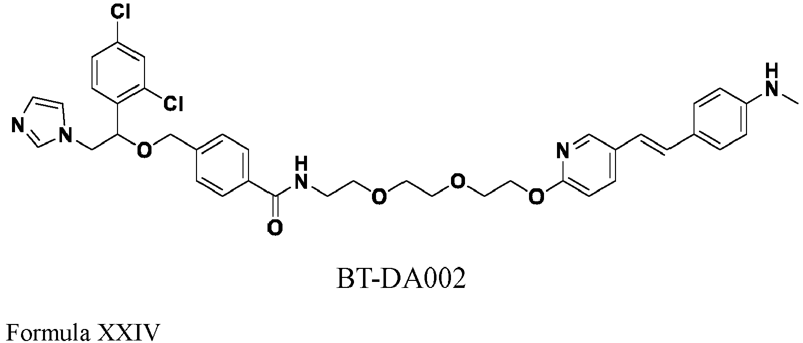

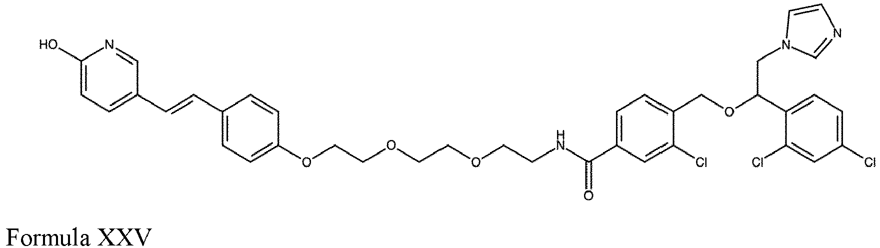

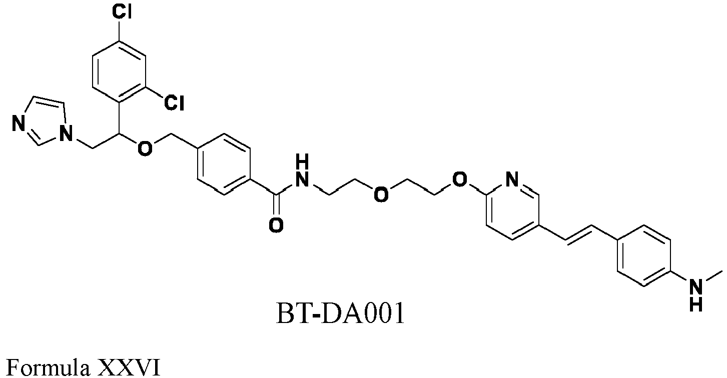

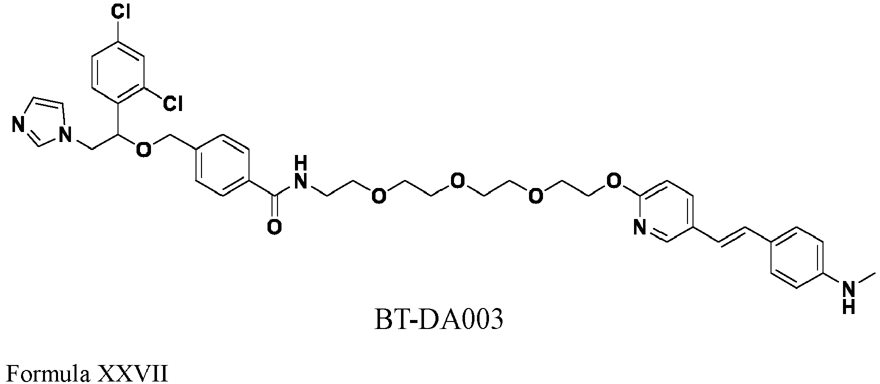

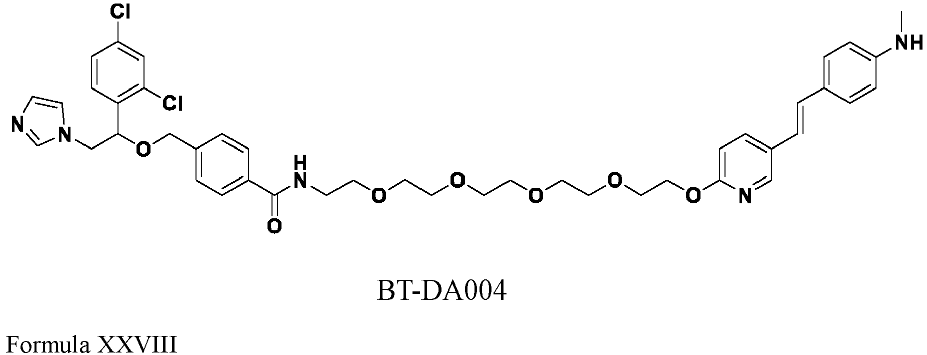

According to a further aspect of the invention, the molecule according to the present invention preferably is bispecific, i.e., has a target molecule binder (B) (or binders to the same target molecule), and a binding moiety stimulating proteasome degradation (S).

Nevertheless, in an alternative embodiment the molecule according to the present invention may additionally comprising at least one moiety having ubiquitin ligase function, such as E3 ligase function, and/or at least one moiety binding to the proteasome 19S subunit. Accordingly, in these cases L usually is a branched linker molecule.

According to a further aspect of the invention, there is a provided a pharmaceutical composition comprising the molecule according to the present invention, and a pharmaceutically or therapeutically acceptable excipient or carrier. Preferably, the pharmaceutical composition comprises a pharmaceutically effective amount of the molecule according to the present invention.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g., lactose, microcrystalline cellulose), disintegrants (e.g., sodium starch glycolate, croscarmellose sodium), binders (e.g. PVP, HPMC), lubricants (e.g., magnesium stearate), glidants (e.g., colloidal SiO₂), solvents/co-solvents (e.g., an aqueous vehicle, Propylene glycol, glycerol), buffering agents (e.g. citrate, gluconates, lactates), preservatives (e.g., Na benzoate, parabens (Me, Pr and Bu), BKC), anti -oxidants (e.g., BHT, BHA, Ascorbic acid), wetting agents (e.g. polysorbates, sorbitan esters), thickening agents (e.g., methylcellulose or hydroxyethylcellulose), sweetening agents (e.g., sorbitol, saccharin, aspartame, acesulfame), flavoring agents (e.g., peppermint, lemon oils, butterscotch, etc.), humectants (e.g., propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention. For example, administration of the pharmaceutical composition as a medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra- atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like. The medicament may be formulated in discrete dosage units and can be prepared by any of the methods well known in the art of pharmacy. The pharmaceutical composition of the present invention can be formulated using methods known in the art to provide rapid, sustained or delayed release of the active ingredient after administration to a mammal.

In addition to the aforementioned molecules of the invention, the pharmaceutical composition can contain two or more molecules according to the present invention and also other therapeutically active substances, depending on the disease or condition to be treated.

The dosage of the pharmaceutical composition according to the present invention can be appropriately selected according to the route of administration, the subject to be administered, the target disease and its severity, age, sex weight, individual differences and disease state. Dosage may be repeated several times a day.

According to the present invention, a subject or patient can be preferably selected from a mammal, such as a mouse, rat, cat, dog, rabbit, goat, sheep, horse, camel, lama, cow, monkey, a farm animal, a sport animal, and a pet, and a human.

According to a further aspect of the invention, there is a provided a method for producing the molecule according to the present invention, comprising suitably linking the at least one binding moiety (B) to the at least one moiety stimulating proteasome degradation (S) through the at least one linker moiety (L). Respective chemistry is known to the person of skill, and described in the art and the present examples.

Preferred is a method for producing the molecule according to the present invention, further comprising the step of identifying the at least one binding moiety (B) and/or the at least one moiety stimulating proteasome degradation (S) before the linking thereof to the at least one linker moiety (L). Thus, the method includes a screening step in order to identify suitable binders (e.g., by screening small molecules libraries, DNA-encoded libraries, peptide libraries, fragment-based libraries, antibody libraries) and/or the moiety stimulating proteasome degradation (e.g., by screening small molecules libraries, DNA-encoded libraries, peptide libraries, fragment-based libraries, antibody libraries). Small molecules have been discovered to stimulate the 20S core particle (CP) of the proteasome to degrade proteins, such as a-synuclein (R. A. Coleman and D. J. Trader, A sensitive high-throughput screening method for identifying small molecule stimulators of the core particle of the proteasome, Curr. Protoc. Chem. Biol., 2018, 10(4), e52, DOI: 10.1002/cpch.52.), and the respective screening method(s) can be readily adapted to the present invention. Preferably, a library of small chemical compounds is screened.

A screening step may also be added to the method in order to identify improved molecules according to the invention, where first the molecules is chemically modified, e.g., by adding or deleting chemical groups, such as adding -COOH, and then the activity as well as other pharmacological properties (e.g., stability, half-life or solubility) are tested. Such optimized molecules are then used to produce the molecules of the invention.

Preferably, the methods according to the present invention are amenable to automation, and are preferably performed in an automated and/or high-throughput format. Usually, this involves the use of chips and respective machinery, such as robots. Automation is particularly preferred in case of the identification of binders and stimulators and/or screening.

Further provided is a molecule of the invention as defined herein for use in the prevention and/or treatment of conditions and/or diseases in a mammalian subject, such as a human. A condition and/or disease suitable for treatment according to the relevant aspects of the invention is one which is characterized by the presence and/or accumulation of undesired (usually proteinaceous) molecules that may be reduced or removed from the cell by proteasomal degradation.

The invention further encompasses the use of a molecule of the invention as an inducer/stimulator and booster of proteasomal degradation. The invention further encompasses the use of a molecule of the invention or the pharmaceutical composition of the invention for improving proteasome function. The invention further encompasses the use of a molecule of the invention or the pharmaceutical composition of the invention for removing an undesired proteinaceous target molecule. The uses may be a cosmetic use and/or in vivo and/or in vitro, for example in an in vitro assay.

Modified or altered proteasomal degradation has been shown to be relevant in neurodegenerative disease, as demonstrated by the accumulation of protein aggregates, for example in Alzheimer disease, Parkinson's disease, polyglutamine diseases, and muscular diseases, and amyotrophic lateral sclerosis (ALS).

Therefore, molecules according to the present invention are for use in the prevention and/or treatment of diseases or conditions in a mammalian subject such as a human selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing,

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress. Amelioration includes in particular the removal of an undesired protein through proteasomal degradation according to the invention.

Molecules according to the present invention are preferably for use in the prevention and/or treatment of diseases or conditions in a mammalian subject such as a human selected from the group consisting of proteopathies related to the following target molecules (in brackets). Alzheimer's disease (Amyloid, Tau), Frontotemporal Dementia (Tau), Progressive supranuclear palsy (Tau), Pick's disease (Tau), Parkinson's disease (alpha-synuclein), Dementia with Lewy bodies (alpha-synuclein), Multiple system atrophy (alpha-synuclein), Amyotrophic Lateral Sclerosis (SOD1, TDP-43, FUS), Huntington's disease (huntingtin), Spinocerebellar ataxias (ataxin), Prion diseases (prions), Cystic fibrosis (CTFR), Alpha 1 antitrypsin deficiency, (Alpha 1 antitrypsin), Diabetes Type-2 (IAPP), Retinitis Pigmentosa (Rhodopsin), Cataracts (Crystallin), Amyloidosis (Amyloid, Transthyretin), and Desmin-related cardiomyopathy (Crystallin).

The target molecule may be advantageously selected from an extracellular protein, since the 20S proteasome may be located in the circulation (extracellular) of an organism. This use is not available in the context of PROTACs.

Further preferred is the molecule for use according to the present invention, wherein said prevention and/or treatment comprises a combination of at least two molecules for use according to the present invention, and/or a combination with at least one additional pharmaceutically active substance for said undesired protein-related disease or condition.

It is to be understood that the present molecule and/or a pharmaceutical composition comprising the present molecule is for use to be administered to a human patient. The term "administering" means administration of a sole therapeutic agent or in combination with another therapeutic agent. It is thus envisaged that the pharmaceutical composition of the present invention is employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e., directly and/or indirectly, preferably synergistically) with the present molecule(s) (for use).

In another aspect thereof, the present invention provides a method for treating or preventing a disease or condition in a mammalian subject, such as a human, comprising administering to a subject in need of said treatment or prevention an effective amount of the molecule according to the present invention or the pharmaceutical composition according to the present invention, wherein the disease or condition to be treated or prevented is selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing.

As above for the molecules for use, the dosage of the pharmaceutical composition to be administered according to the present invention can be appropriately selected according to the route of administration, the subject to be administered, the target disease and its severity, age, sex weight, individual differences and disease state. Dosing may be repeated several times a day.

According to the present invention, a mammalian subject or patient can be preferably selected from a mouse, rat, cat, dog, rabbit, goat, sheep, horse, camel, lama, cow, monkey, a farm animal, a sport animal, a pet, and a human.

In addition to the aforementioned molecules of the invention, the pharmaceutical composition as administered can contain two or more molecules according to the present invention and also other therapeutically active substances. In the method, the molecules for use can be provided and/or is administered as a suitable pharmaceutical composition as discussed above. The molecules can be administered alone or in combination with other active molecules or compounds - for example with medicaments already known for the treatment of the aforementioned conditions and/or diseases, whereby in the latter case a favorable additive, amplifying or preferably synergistically effect is noticed.

It was surprisingly found that the molecules according to the invention were shown to be surprisingly effective inducers/stimulators of the proteasome, and even synergistically effective (boosters). Preferred is thus the molecule according to the present invention, wherein said molecule synergistically stimulates/improves the degradation of the proteinaceous target molecule as disclosed herein. In comparison with prior art compounds as tested (including data not shown), an improvement of the present invention lies in the unexpected observation that the compounds described herein are highly effective inducers/stimulators of the proteasome (see Examples below).

The present invention will now be described further in the following examples with reference to the accompanying Figures, nevertheless, without being limited thereto. For the purposes of the present invention, all references as cited herein are incorporated by reference in their entireties. In the Figures,

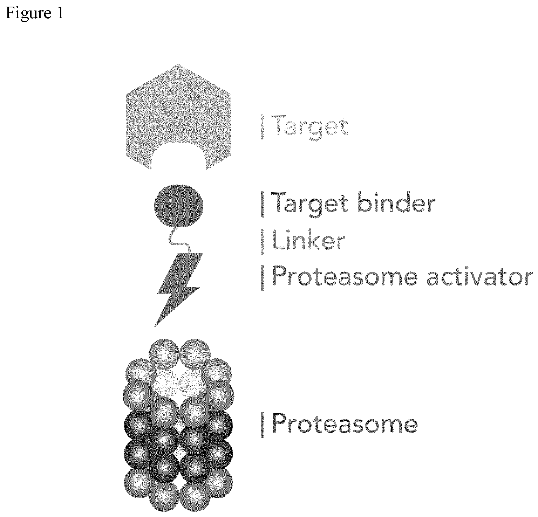

Figure 1 shows a schematic overview of the function and structure of the molecules according to the present invention.

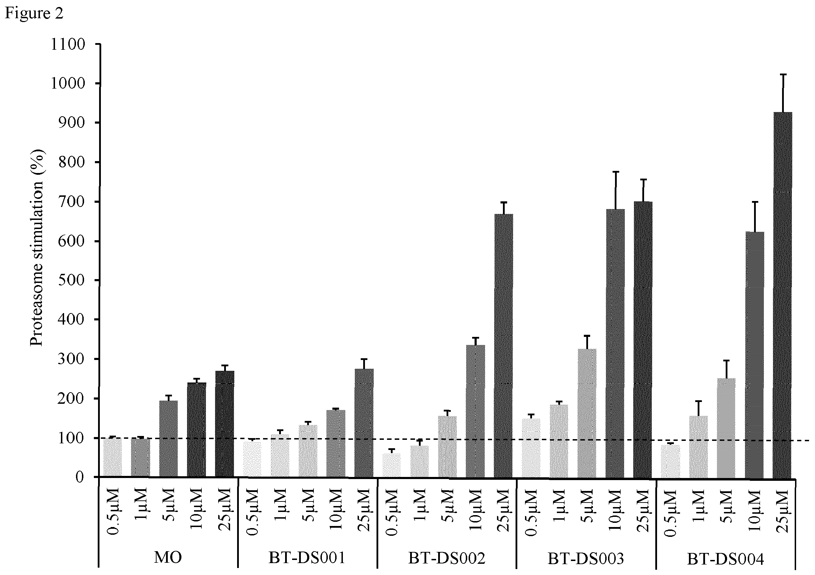

Figure 2 shows the results of the functional assay for the compounds of group I, based on proteasome stimulation *in vitro.* The biochemical assay was performed with a purified human 20S proteasome. The compounds were incubated with 20S proteasomes for 1h, and the proteasome activity was measured with a TAS-1 probe. DMSO samples were used as control (dashed line).

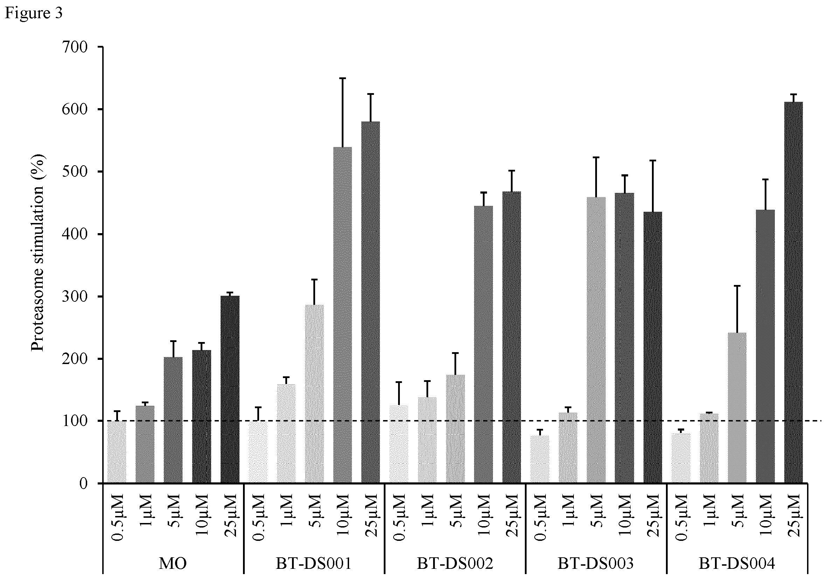

Figure 3 shows the results of the assay for proteasome activity in HEK293 cells for the compounds of group I. The compounds were dosed for 1.5 h, the proteasome activity was measured with Me4BodipyFLAhx3L3VS activity probe, DMSO samples were used as control (dashed line).

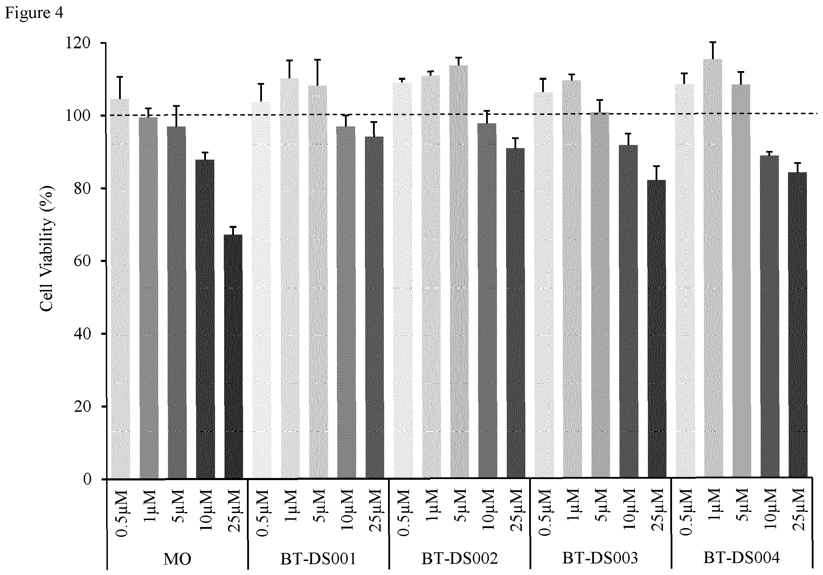

Figure 4 shows the results of the assay for cellular toxicity in HEK293 cells for the compounds of group I. The compounds were dosed for 24h, the viability measured with CellTiter-Glo, and DMSO was used as control (dashed line).

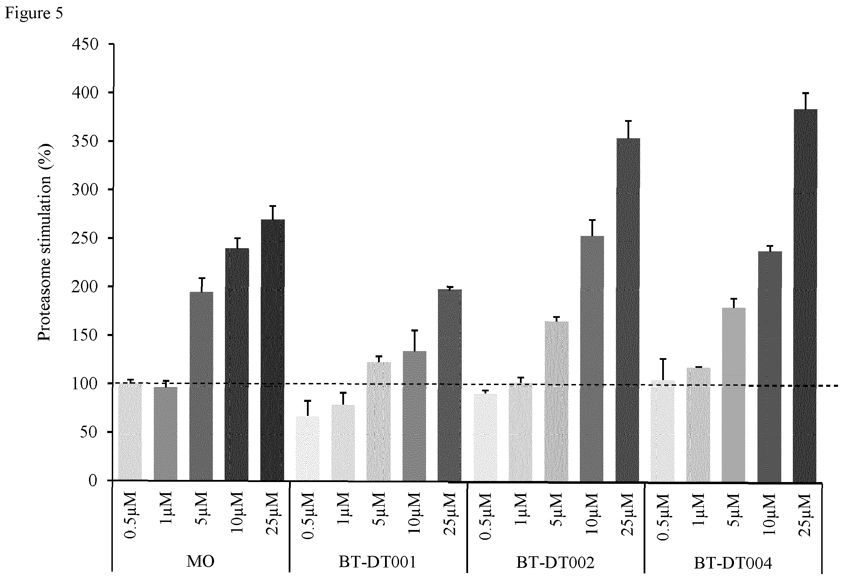

Figure 5 shows the results of the functional assay for the compounds of group II, based on proteasome stimulation *in vitro.* The biochemical assay was performed with a purified human 20S proteasome. The compounds were incubated with 20S proteasomes for 1h, and the proteasome activity was measured with a TAS-1 probe. DMSO samples were used as control (dashed line).

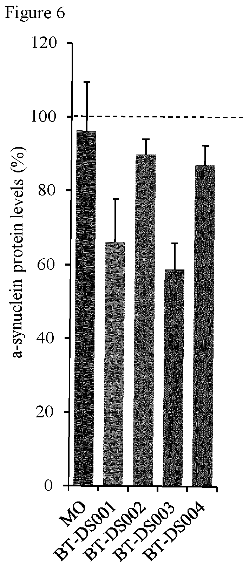

Figure 6 shows the results of the functional assay for the compounds of group I, a-synuclein degradation in HEK293 cells. After a-synuclein transfection for 48h, the compounds were dosed for 3h at 25µM together with 50µM cycloheximide, and the total a-synuclein was determined using western blot. DMSO samples were used as control (dashed line).

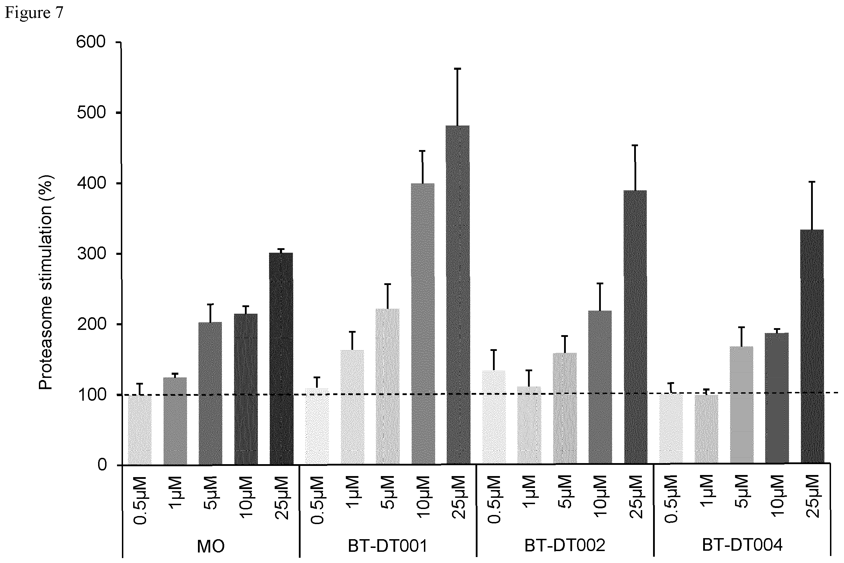

Figure 7 shows the results of the functional assay for the compounds of group II, proteasome stimulation in cells. For proteasome activity in HEK293 cells, the compounds were dosed for 1.5h, and the proteasome activity measured with Me4BodipyFLAhx3L3VS activity probe. DMSO samples were used as control (dashed line).

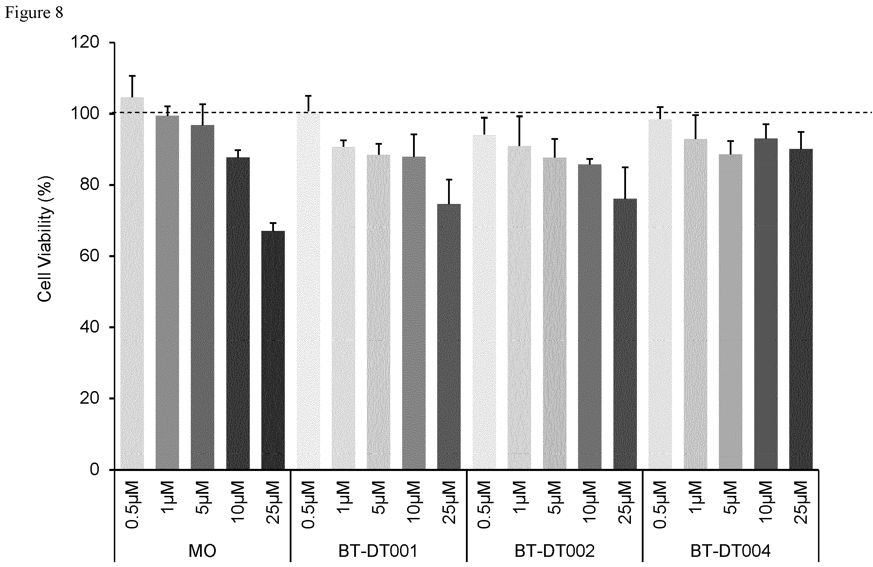

Figure 8 shows the results of the cell toxicity assay in HEK293 cells for the compounds of group II. Compounds were dosed for 24h, and the viability measured with CellTiter-Glo. DMSO samples were used as control (dashed line).

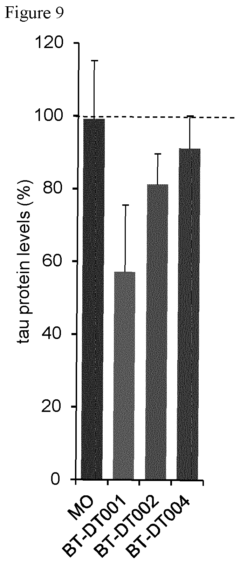

Figure 9 shows the results of the functional assay for the compounds of group II, tau degradation in HEK293 cells. After tau transfection for 48h, the compounds were dosed for 16h at 25µM together with 30µM cycloheximide, and total tau was determined (western blot). DMSO samples were used as control (dashed line). Preferred compound DT-001 shows clear tau degradation improvement.

### EXAMPLES

In the context of the present invention, the following molecules were produced (synthesized), and tested. The molecules as a preferred embodiment comprise miconazole as a proteasome stimulator (Trader DJ, RSC Chemical Biology, 2021), (PEG)ₓ with x selected from 3 to 5 as a preferred linker, and a specific target binder (here thioflavin, or flortaucipir, or florbetapir). The compounds here described are designed to deliver a protein of interest to the proteasome for degradation while boosting proteasome activity.

### I. a-Synuclein degradation molecules

The molecules of this group are composed of miconazole as the activator, (PEG)x as the ligand, and thioflavin as well as derivatives thereof as the target binder. These compounds are thus designed to promote the degradation of a-synuclein with enhanced proteasome activity.

### 1. Miconazole (stimulator)

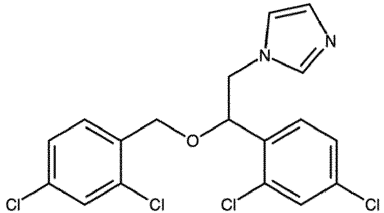

### 2. (PEG)ₓ - linker sequences

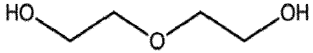

or

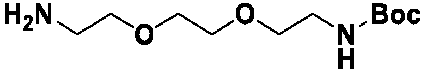

### 3. Thioflavin - target binder

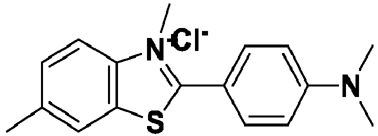

Thioflavins (ThT) are fluorescent dyes used to monitor protein aggregation binding to monomers, oligomers, and fibrils (from a-synuclein), prevent protein aggregation, and extend lifespan (Alavez, S., Vantipalli, M., Zucker, D. et al. Amyloid-binding compounds maintain protein homeostasis during ageing and extend lifespan. Nature 472, 226-229 (2011). https://doi.org/10.1038/nature09873; Romani M, Sorrentino V, Oh CM, Li H, de Lima TI, Zhang H, Shong M, Auwerx J. NAD+ boosting reduces age-associated amyloidosis and restores mitochondrial homeostasis in muscle. Cell Rep. 2021 Jan 19;34(3):108660. doi: 10.1016/j.celrep.2020.108660. PMID: 33472069; PMCID: PMC7816122). Recently, a PROTAC was developed with the ThT backbone to degrade a-synuclein (WO2020041331A1).

### Group I: Compounds according to the present invention

The compounds of Group I were generally synthesized as follows:

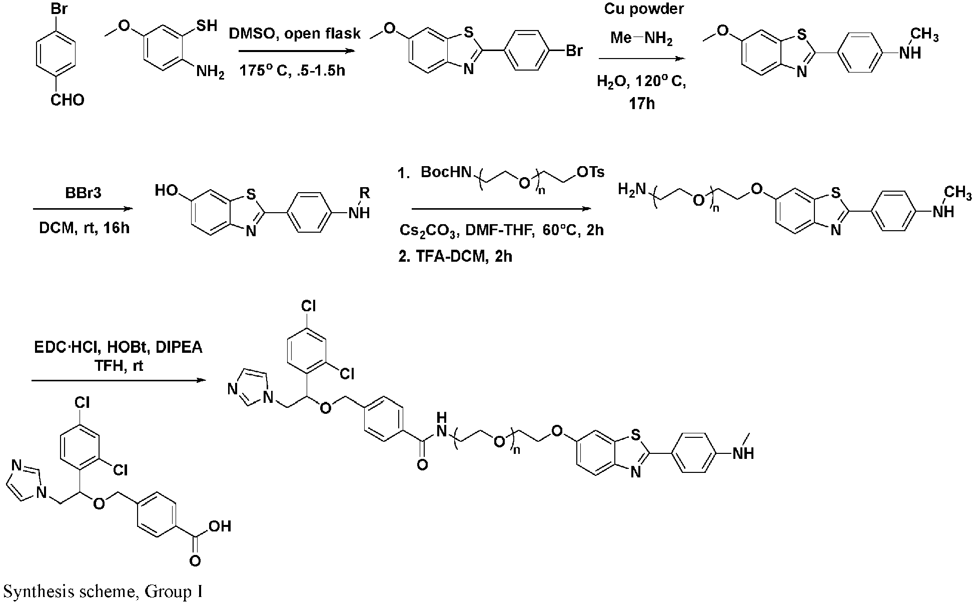

### Compounds Group I:

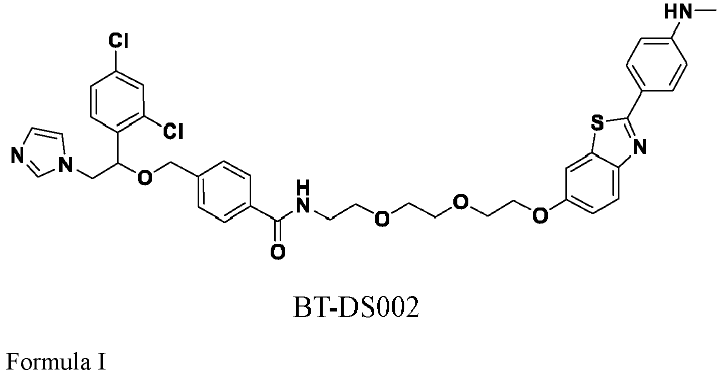

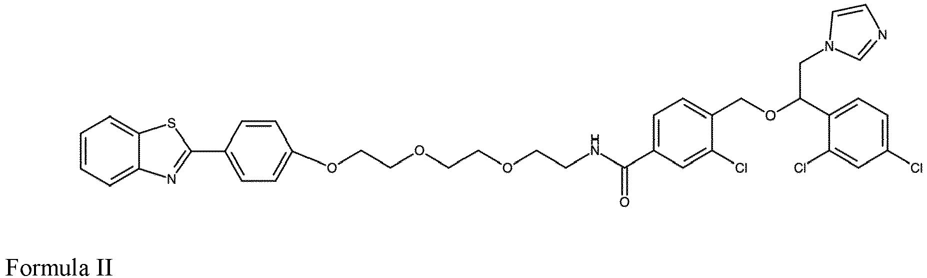

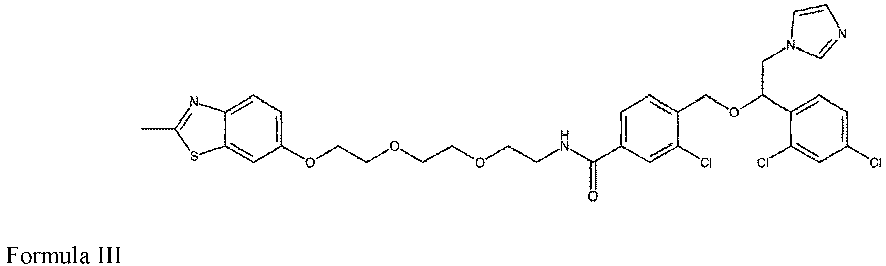

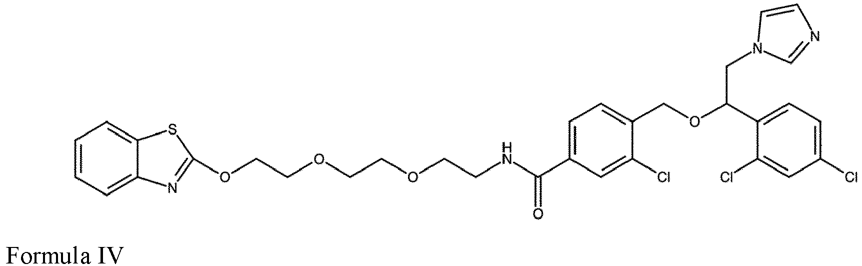

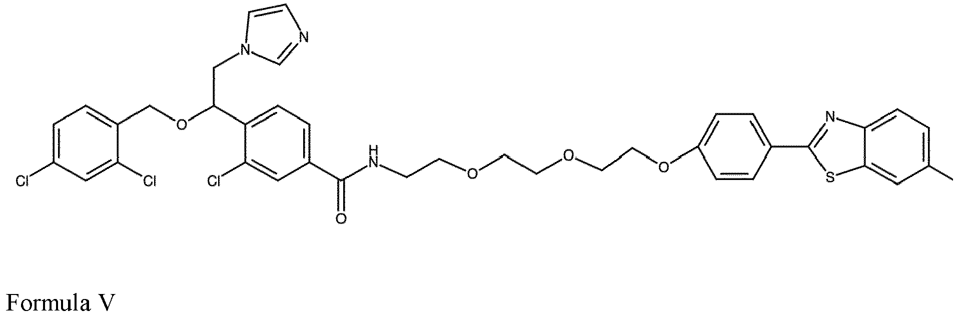

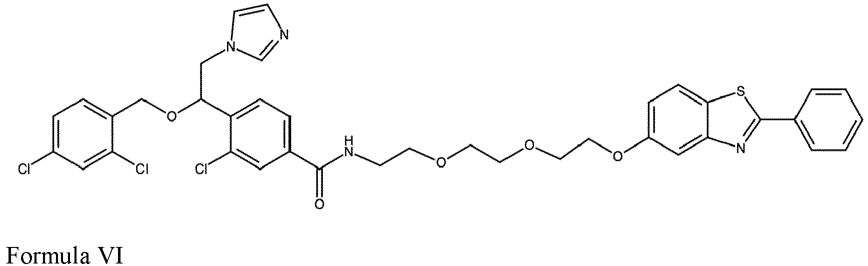

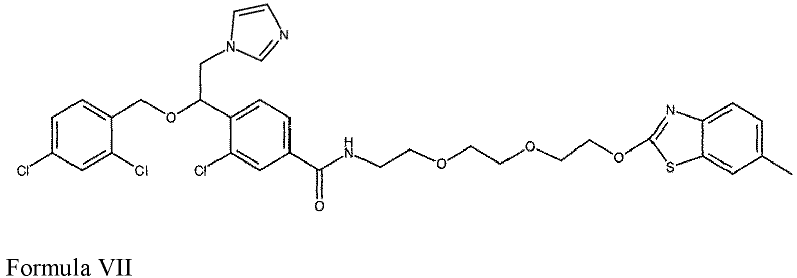

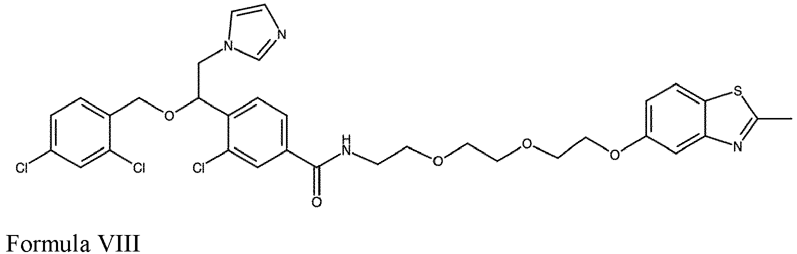

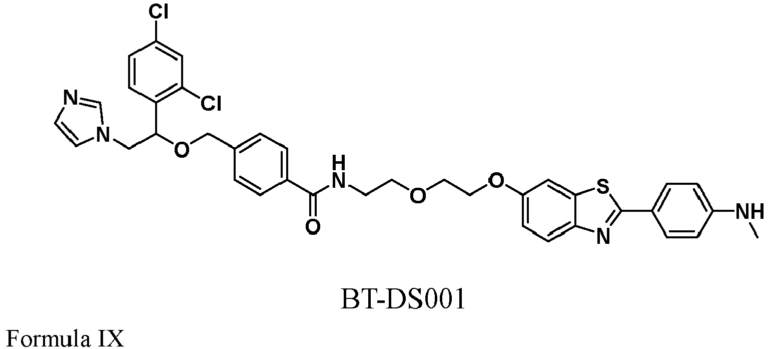

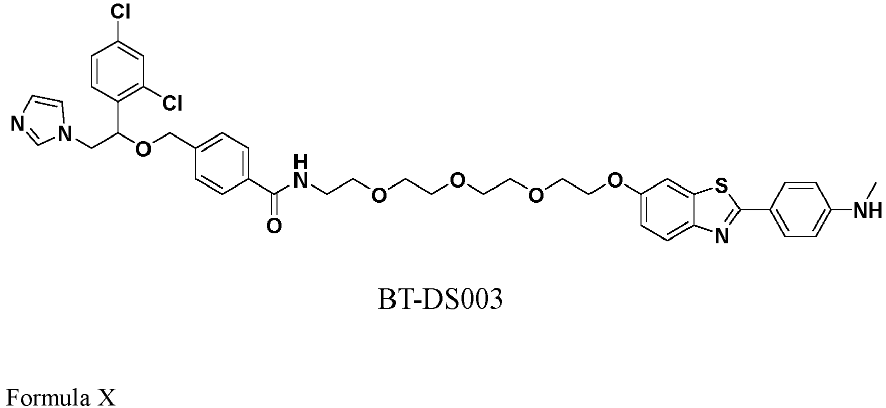

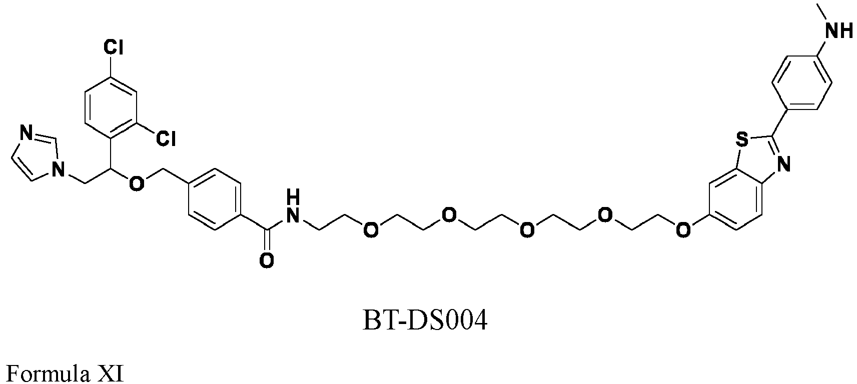

Figures 2 to 4 show the results of the functional assay for the compounds of group I, Figure 2 based on proteasome stimulation *in vitro.* The biochemical assay was performed with a purified human 20S proteasome. The compounds were incubated with 20S proteasomes for 1h, and the proteasome activity was measured with a TAS-1 probe. DMSO samples were used as control. Figure 3 shows the results of the assay for proteasome activity in HEK293 cells for the compounds of group I. The compounds were dosed for 1.5 h, the proteasome activity was measured with Me4BodipyFLAhx3L3VS activity probe, DMSO samples were used as control. Figure 4 shows the results of the assay for cellular toxicity in HEK293 cells for the compounds of group I. The compounds were dosed for 24h, the viability measured with CellTiter-Glo, and DMSO was used as control (dashed line). The inventive compounds are better stimulators than the activator alone (MO), especially when they have a longer linker, e.g. (PEG)₄₋₅. In particular, the degraders DS001 and DS003 show a clear improvement in degrading a-synuclein (compared to DMSO and MO), see Figure 6.

### II. Tau degrader and proteasome supercharger

The molecules of this group are composed of miconazole as the activator, (PEG)x as the ligand, and Flortaucipir as well as derivatives thereof as the target binder. These compounds are thus designed to promote the degradation of tau with enhanced proteasome activity.

### 1. Miconazole (stimulator)

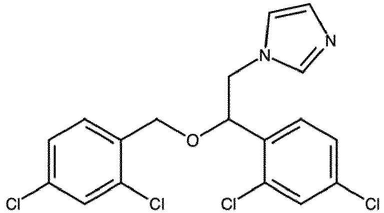

### 2. (PEG)ₓ - linker sequences

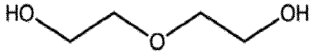

or

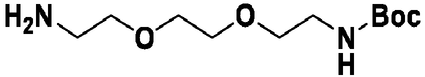

### 3. Flortaucipir (18F)- target binder

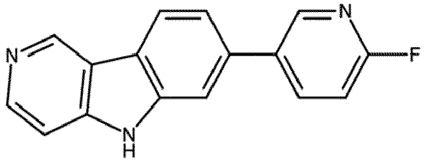

Flortaucipir 18F (Chun-Fang Xia, et al. [18F] T807, a novel tau positron emission tomography imaging agent for Alzheimer's disease, Alzheimer's & Dementia, Volume 9, Issue 6, 2013, Pages 666-676, ISSN 1552-5260, https://doi.org/10.1016/j.jalz.2012.11.008) is an FDA approved PET agent to monitor tau levels (Eli Lilly product Tauvid). Tauvid binds tau *in vivo* and preferentially binds to mutated tau forms associated with Alzheimer's disease pathology (David T. Jones, et al. In vivo 18F-AV-1451 tau PET signal in MAPT mutation carriers varies by expected tau isoforms, Neurology Mar 2018, 90 (11) e947-e954; DOI: 10.1212/WNL.0000000000005117). Recently, a PROTAC was developed with the Flortaucipir backbone to degrade tau (WO 2019/014429).

### Group II: Compounds according to the present invention

The compounds of group II were generally synthesized as follows:

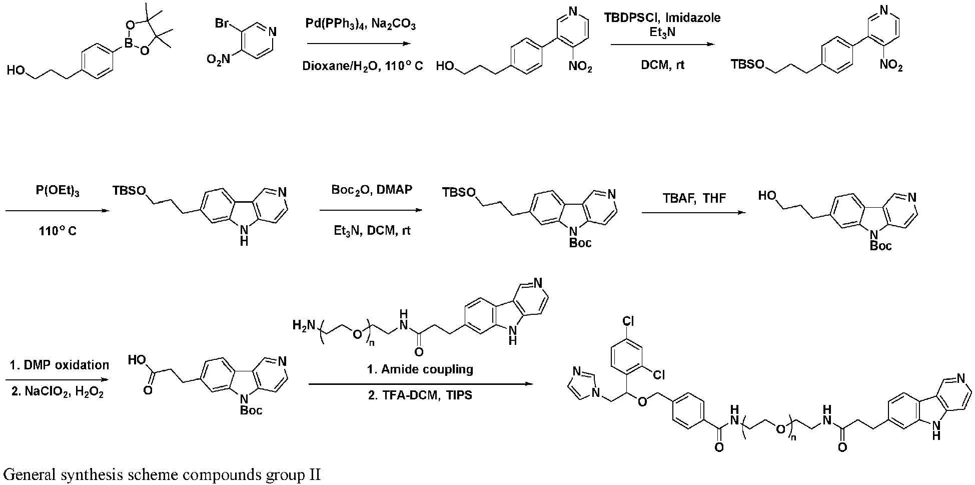

### Compounds of group II

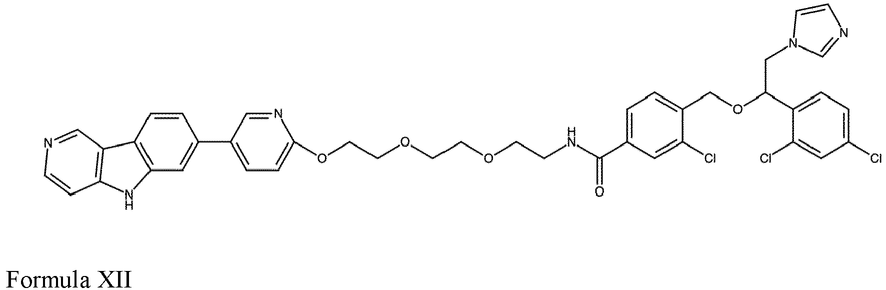

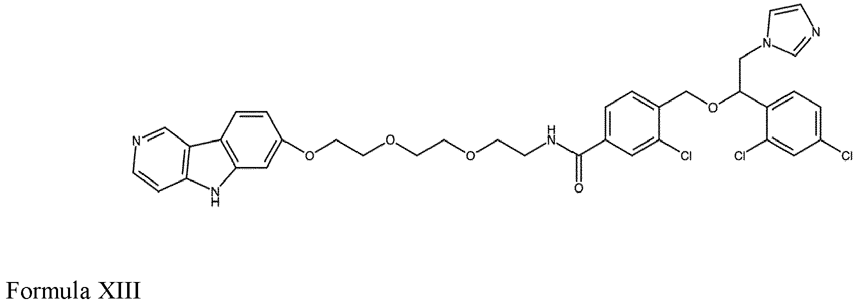

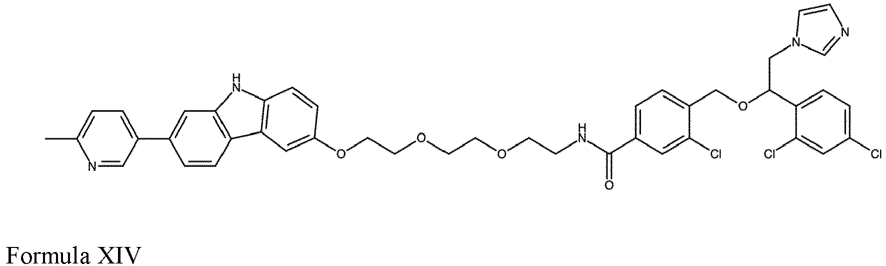

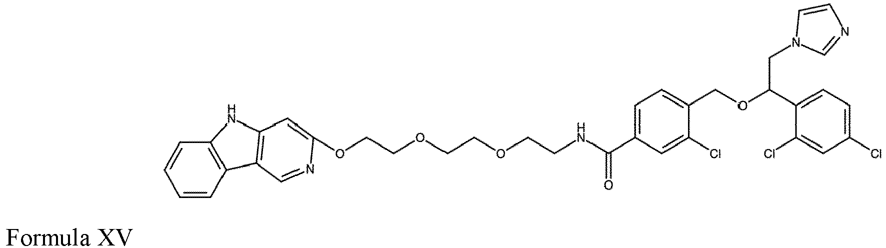

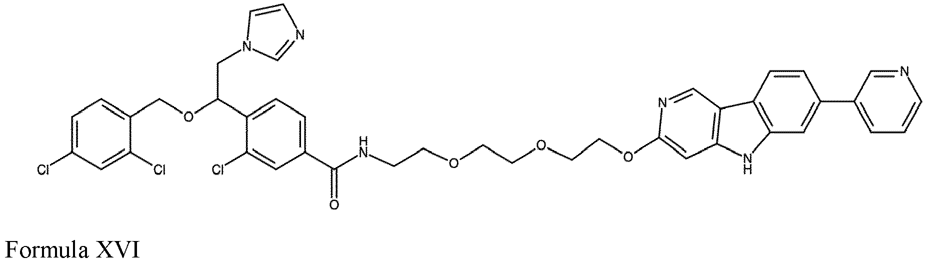

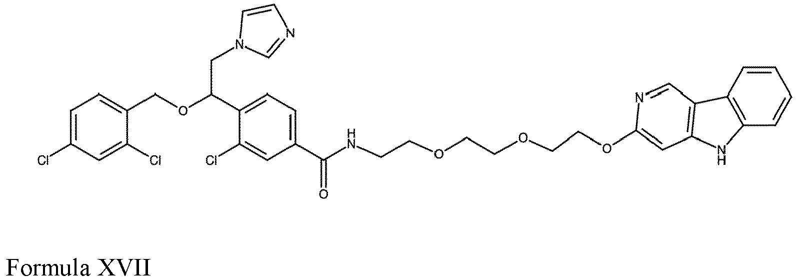

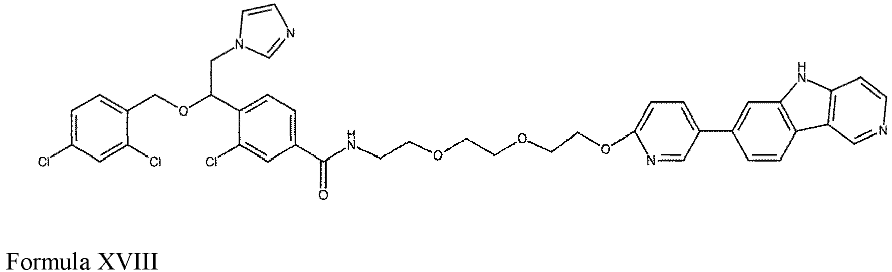

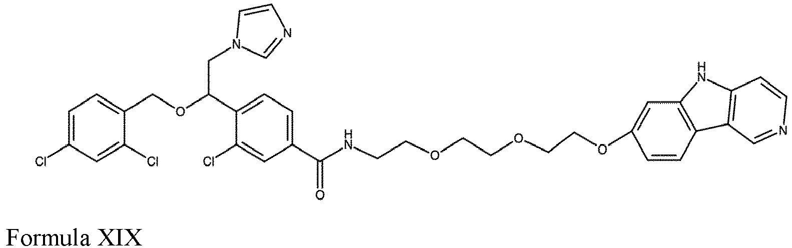

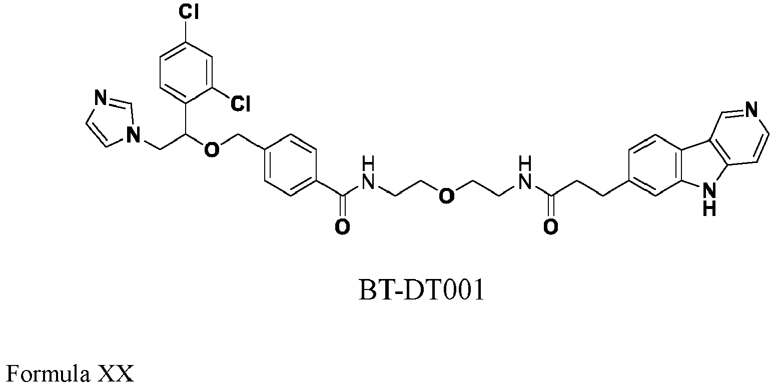

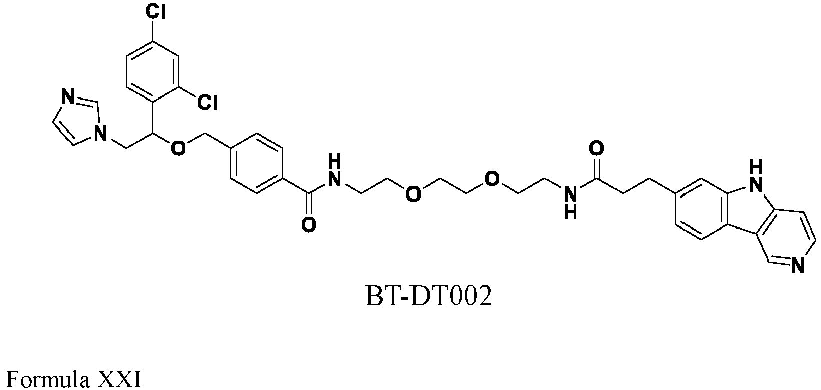

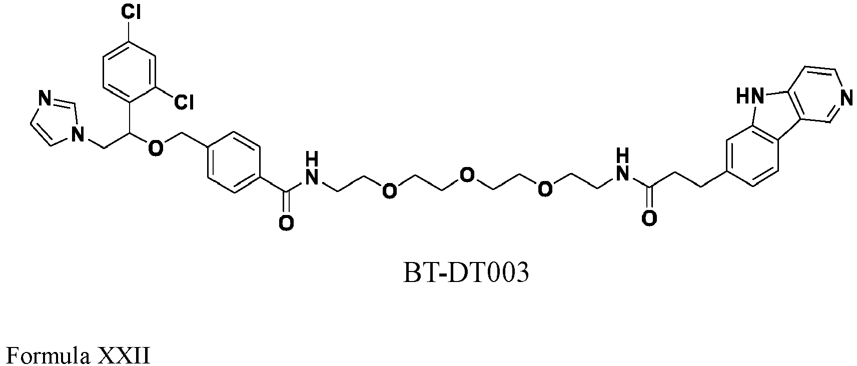

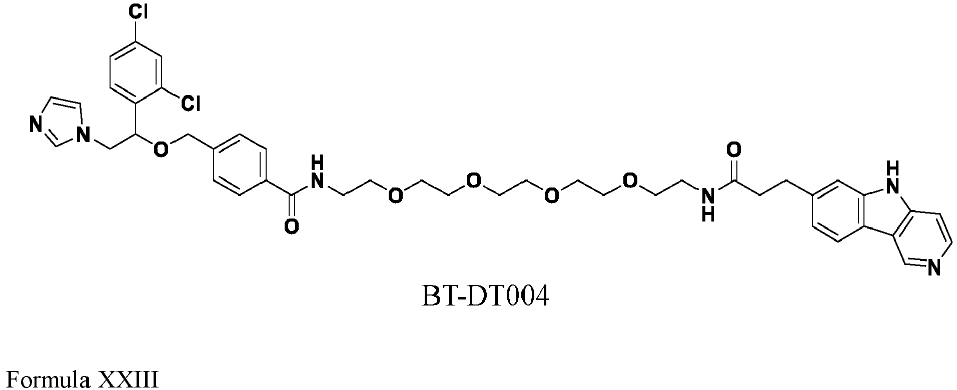

Figure 5 shows the results of the functional assay for the compounds of group II, based on proteasome stimulation *in vitro.* The biochemical assay was performed with a purified human 20S proteasome. The compounds were incubated with 20S proteasomes for 1h, and the proteasome activity was measured with a TAS-1 probe. DMSO samples were used as control (dashed line). The inventive compounds are better stimulators than the activator alone (MO), especially when they have a longer linker, e.g., (PEG)₄₋₅. Figure 7 shows the results of the functional assay for the compounds of group II, proteasome stimulation in cells. For proteasome activity in HEK293 cells, the compounds were dosed for 1.5h, and the proteasome activity measured with Me4BodipyFLAhx3L3VS activity probe. DMSO samples were used as control (dashed line). Figure 8 shows the results of the cell toxicity assay in HEK293 cells for the compounds of group II. Compounds were dosed for 24h, and the viability measured with CellTiter-Glo. DMSO samples were used as control (dashed line). Figure 9 shows the results of the functional assay for the compounds of group II, tau degradation in HEK293 cells. After tau transfection for 48h, the compounds were dosed for 16h at 25µM together with 30µM cycloheximide, and total tau was determined (western blot). DMSO samples were used as control (dashed line). Preferred compound DT-001 shows clear tau degradation improvement.

### III. Amyloid degrader and proteasome supercharger

The molecules of this group are composed of miconazole as the activator, (PEG)x as the ligand, and Florbetapir as well as derivatives thereof as the target binder. These compounds are thus designed to promote the degradation of beta-amyloids with enhanced proteasome activity.

### 1. Miconazole (stimulator)

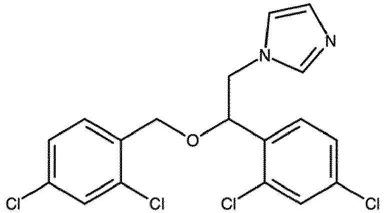

### 2. (PEG)ₓ - linker sequences

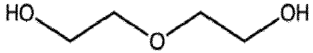

or

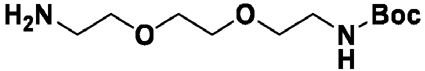

### 3. Florbetapir - target binder

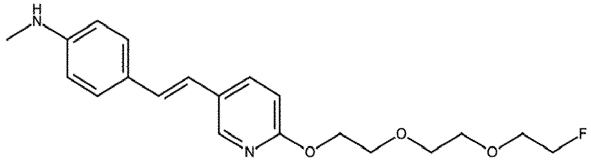

Florbetapir F18 (Wei Zhang, et al. 18F-labeled styrylpyridines as PET agents for amyloid plaque imaging, Nuclear Medicine and Biology, Volume 34, Issue 1, 2007, Pages 89-97, ISSN 0969-8051, https://doi.org/10.1016/j.nucmedbio.2006.10.003) is an FDA approved PET agent to monitor beta-amyloid levels (Eli Lilly product - Amyvid). Amyvid binds beta-amyloid *in vivo* (John Lister-James, et al. Florbetapir F-18: A Histopathologically Validated Beta-Amyloid Positron Emission Tomography Imaging Agent, Seminars in Nuclear Medicine, Volume 41, Issue 4, 2011, Pages 300-304, ISSN 0001-2998, https://doi.org/10.1053/j.semnuclmed.2011.03.001).

The compounds of Group III were generally synthesized as follows.

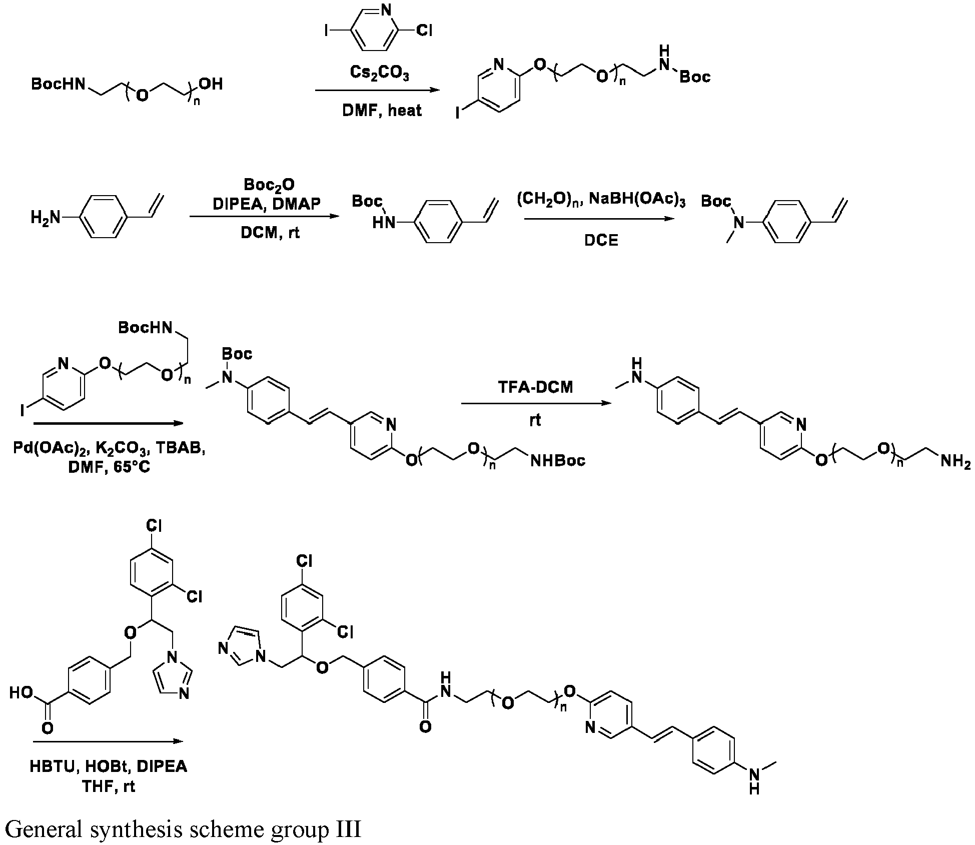

### Group III: Compounds according to the present invention

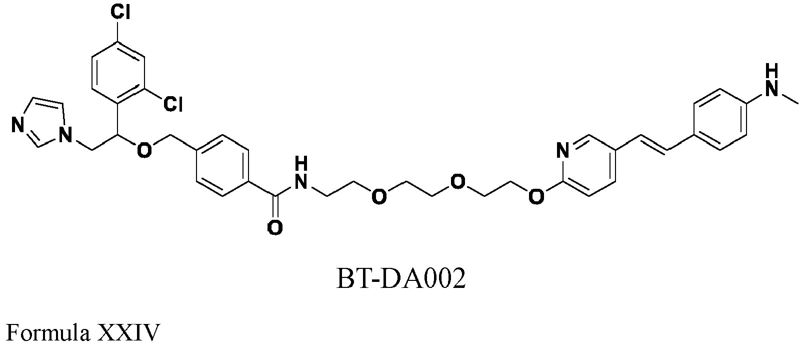

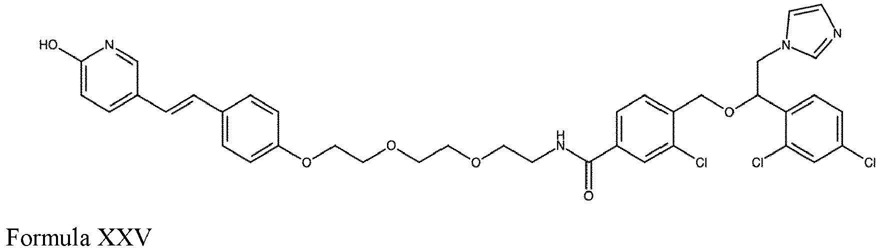

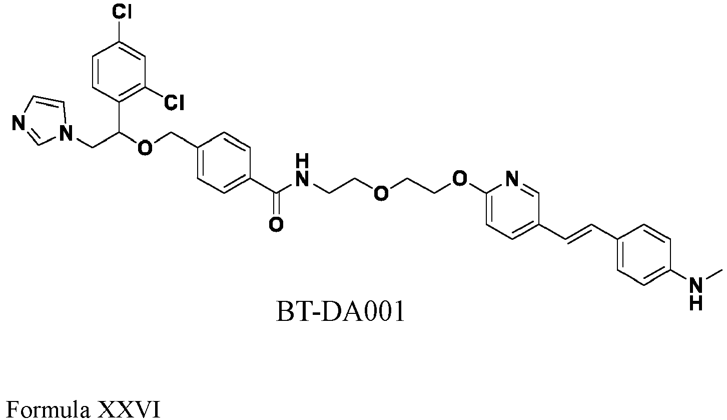

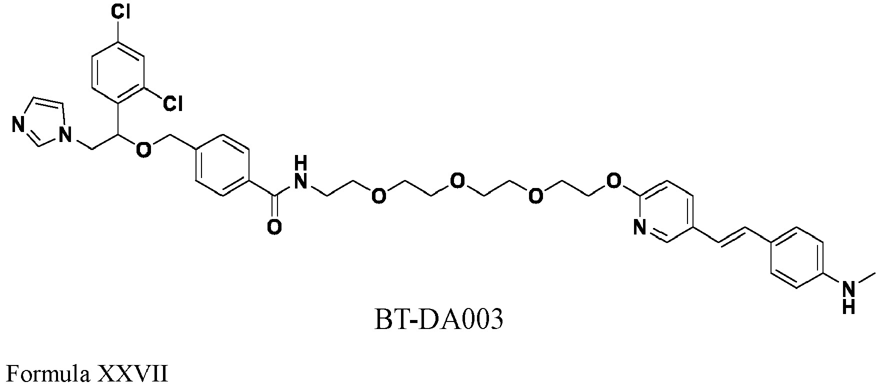

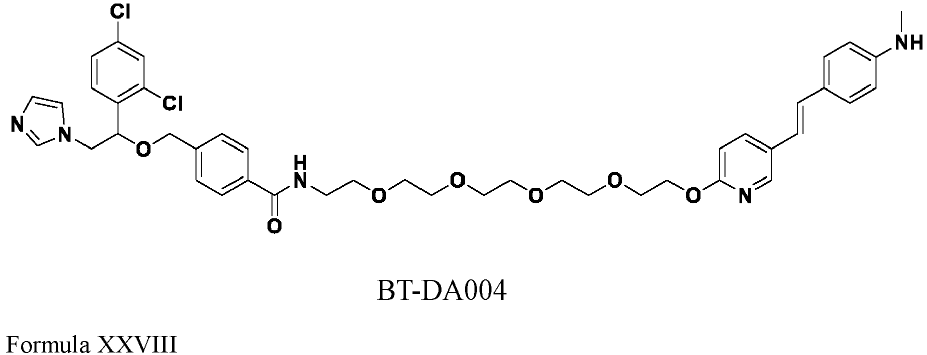

The inventive compounds are better stimulators than the activator alone, especially when they have a longer linker, i.e. (PEG)₅.

## Claims

1. A molecule improving the degradation of a proteinaceous target molecule by the proteasome of a cell, according to the general formula
B―L―S
wherein
B indicates at least one binding moiety binding or promoting binding to the target molecule,
S indicates at least one moiety stimulating proteasome degradation, and
L indicates at least one linker moiety suitably linking said at least one binding moiety with said at least one moiety stimulating proteasome degradation, and
pharmaceutically acceptable salts thereof.

2. The molecule according to claim 1, wherein the target molecule is selected from the group consisting of an accumulated undesired protein, an accumulated pathological protein, prions, beta-amyloid, tau, alpha-synuclein, estrogen receptor alpha (ERα), androgen receptor (AR), huntingtin, ataxin, methionine aminopeptidase-1 (MetAP-2), cellular retinoic acid-binding protein-I, cellular retinoic acid-binding protein-II (CRABP-II), alpha 1 antitrypsin, rhodopsin, crystallin, transthyretin, amyloid, cystic fibrosis transmembrane conductance regulator (CTFR), superoxide dismutase 1 (SOD1), transactive response DNA binding protein 43 kDa (TDP-43), fused in sarcoma (FUS), and islet amyloid polypeptide (IAPP).

3. The molecule according to claim 1 or 2, wherein the at least one binding moiety (B) binding or promoting binding to the target molecule is selected from the group consisting of an antibody that is specific for said target molecule or a specific binding fragment thereof, a proteinaceous group specifically binding to said target molecule, a binding peptide, a natural or non-natural nucleic acid, such as an aptamer, and a small molecule specifically binding to said target molecule, florbetapir F18, amyvid, tauvid, and thioflavin.

4. The molecule according to any one of claims 1 to 3, wherein the at least one binding moiety stimulating proteasome degradation (S) is selected from the group consisting of a compound stimulating degradation in an ubiquitin-independent and/or dependent manner, a compound stimulating the 20S core particle (CP) of the proteasome, miconazole, and derivatives thereof.

5. The molecule according to any one of claims 1 to 4, wherein the at least one linker moiety (L) suitably linking said at least one binding moiety with said at least one moiety stimulating proteasome degradation is selected from the group consisting of a branched or unbranched chemical linker compound, a branched or unbranched linker amino acid sequence, a cleavable branched or unbranched linker compound, and a labelled branched or unbranched linker compound, in particular a polyethylene linker sequence, such as (PEG)₃.

6. The molecule according to any one of claims 1 to 5, selected from the group of the compounds according to any one of formulae I to XXVIII, and pharmaceutically acceptable salts and derivatives thereof.

7. The molecule according to any one of claims 1 to 6, wherein said molecule is bispecific.

8. The molecule according to any one of claims 1 to 7, wherein said molecule synergistically improves the degradation of the proteinaceous target molecule.

9. The molecule according to any one of claims 1 to 6 and 8, additionally comprising at least one moiety having ubiquitin ligase function, such as E3 ligase function, and/or at least one moiety binding to the proteasome 19S subunit.

10. A method for producing a molecule according to any one of claims 1 to 9, comprising suitably linking the at least one binding moiety (B) to the at least one moiety stimulating proteasome degradation (S) through the at least one linker moiety (L).

11. The method according to claim 10, further comprising the step of identifying the at least one binding moiety (B) and/or the at least one moiety stimulating proteasome degradation (S) before the linking thereof to the at least one linker moiety (L).

12. A pharmaceutical composition, comprising the molecule according to any one of claims 1 to 9 or produced according to claim 10 or 11, or a pharmaceutically acceptable salt thereof, together with at least one pharmaceutically acceptable carrier or buffer.

13. The molecule according to any one of claims 1 to 9 or the pharmaceutical composition according to claim 12 for use in the prevention and/or treatment of diseases or conditions in a mammalian subject, such as a human.

14. The molecule or the pharmaceutical composition for use according to claim 13, wherein the diseases or conditions to be treated or prevented are selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing.

15. Use of the molecule according to any one of claims 1 to 9 or the pharmaceutical composition according to claim 12 for improving proteasome function.

16. Use of the molecule according to any one of claims 1 to 9 or the pharmaceutical composition according to claim 12 for removing an undesired proteinaceous target molecule.

17. A method for treating or preventing a disease or condition in a mammalian subject, such as a human, comprising administering to a subject in need of said treatment or prevention an effective amount of the molecule according to any one of claims 1 to 9 or the pharmaceutical composition according to claim 12, wherein the disease or condition to be treated or prevented is selected from the group consisting of a disease or condition caused by an undesired proteinaceous target molecule, caused by an accumulated pathological protein, proteopathies, alpha-synucleinopathies, tauopathies, neurodegenerative diseases associated with alpha-synuclein accumulation or aggregation, neurodegenerative diseases associated with tau accumulation or aggregation, neurodegenerative diseases associated with beta-amyloid accumulation or aggregation, Parkinson's disease, Alzheimer's disease, dementia, dementia with Lewy bodies, frontotemporal dementia, progressive supranuclear palsy, Pick's disease, amyotrophic lateral sclerosis, Huntington's disease, spinocerebellar ataxias, prion diseases, cystic fibrosis, alpha 1 antitrypsin deficiency, diabetes type-2, retinitis pigmentosa, cataracts, amyloidosis, desmin-related cardiomyopathy, multiple system atrophy, cancer, breast cancer, prostate cancer, cancer tumor metastasis, and ageing.
